# EUROPEAN PATENT SPECIFICATION

(11) **EP 3 165 087 B1**
(45) Date of publication and mention of the grant of the patent: **22.07.2020**
(21) Application number: 16176713.2
(22) Date of filing: 23.10.2012
(51) Int. Cl.: A01K 67/033

(54) **MITE COMPOSITION, CARRIER, METHOD FOR REARING MITES AND USES RELATED THERETO**
MILBENZUSAMMENSETZUNG, TRÄGER, VERFAHREN ZUR AUFZUCHT VON MILBEN UND DAMIT VERBUNDENE VERWENDUNGEN
COMPOSITION POUR MITE, SUPPORT, PROCÉDÉ D'ÉLEVAGE DE MITES ET UTILISATIONS ASSOCIÉES

(30) Priority: 04.01.2012 US 201261583150 P
(43) Date of publication of application: 10.05.2017
(62) Divisional of application: 12783358.0
(73) Proprietor: Koppert B.V., 2651 BE Berkel en Rodenrijs (NL)
(72) Inventor: BOLCKMANS, Karel Jozef Florent, 2320 Hoogstraten (Wortel) (BE); VAN HOUTEN, Yvonne Maria, 2671 PG Naaldwijk (NL); VAN BAAL, Adelmar Emmanuel, 2614 TH Delft (NL); TIMMER, Radbout, 2593 PL Den Haag (NL); MOREL, Damien Marc, 2651 BE Berkel en Rodenrijs (NL)
(74) Representative: Patentwerk B.V.

(56) References cited:
- EP-A1- 0 777 963
- WO-A1-97/34468
- WO-A1-2006/057552
- WO-A1-2006/071107
- WO-A1-2007/075081
- Marilyn Y Steiner ET AL: "Methods for collecting and rearing thrips (Thysanoptera) and their natural enemies", AUSTRALIAN JOURNAL OF ENTOMOLOGY, vol. 37, no. 2, 1 June 1998 (1998-06-01), pages 101-106, XP055532421, AU ISSN: 1326-6756, DOI: 10.1111/j.1440-6055.1998.tb01554.x
- PRADO FREIRE; MORAES: "Mass production of the predatory mite Stratiolaelaps scimitus (Womersley) (Acari: Laelapidae)", SYSTEMATIC AND APPLIED ACAROLOGY, vol. 12, no. 2, 30 September 2007 (2007-09-30), pages 117-119, ISSN: 1362-1971

## Description

The present invention in general relates to the field of rearing of commercially relevant mites. More particularly the present invention relates to a mite composition, suitable for the commercial rearing of mites, a method for rearing mites and a rearing device using the composition of the invention, a method for crop protection using the composition according to the invention wherein the mite is selected as a predatory mite, and the use of a carrier material for rearing a mite species.

During the past years the commercial interest in mites has increased. For example the use of predatory mites for biological crop protection is becoming increasingly popular in agriculture. Currently *Phytoseiid* predatory mites are employed to combat pests such as *phytophagous mites, thrips* and whiteflies. In addition other predatory mite species selected from *Mesostigmatid* and *Prostigmatid* predatory species, such as from the family of the *Macrochelidae, Laelapidae, Cheyletidae, Parasitidae, Tydeidae, Cunaxidae, Erythraeidae* receive attention in biological pest control and some have entered the market.

A driving force behind the popularity of predatory mites is their efficacy to control harmful crop pests and the availability of mass rearing systems to produce them on a commercially relevant scale for an acceptable price. This enables the use of predatory mites as an economic alternative to chemical pesticides. In the present commercial rearing systems populations of the predatory mites are reared on life prey in a culture maintained on a carrier.

Such mass rearing systems for predatory mites depend heavily on the availability of suitable prey for the predators. In view of this, during the past years, there have been major efforts in providing rearing prey (or alternatively rearing hosts) for predatory mites. Especially mites from the family of the *Astigmata* have been identified as such suitable rearing prey (see for example WO2006/057552, WO2006/071107, WO2007/075081, WO2008/015393, WO2008/104807 and EP2232986). In view of their role in rearing of predatory mites, the commercial relevance of rearing preys is increasing. WO97/34468 relates to a method for breeding entomophagous insects or mites using popcorn grains coated with a food source.

In view of the above there is a continuing need to improve rearing systems of both predatory mites and mites suitable as rearing prey. The inventors of the present invention have now surprisingly found that rearing systems of commercially relevant mites may be improved by selecting a carrier comprising stacked carrier elements selected from millet husks, wherein the carrier elements comprise shelters for mite individuals. Without wishing to be bound by any theory it is believed that in providing shelters for the mite individuals the mite individuals may shelter from disturbing interspecific and/or intraspecific interactions, such as motional activity, disturbance, interference and cannibalism, with other mite individuals. This may in particular be relevant for juvenile life stages, especially at high population densities.

Tests have shown that mites for ovipositing prefer carrier material according to the invention over the non-sheltering carriers presently used in rearing of such mites. This preference may reflect the quality of such carriers for ovipositing to increase the chance of survival and successful development of the eggs and juvenile stages.

According to a first aspect the invention therefore relates to a mite composition according to claim 1 comprising:
- a population of individuals of a mite species, preferably a mite species selected from *Mesostigmatid* predatory mite species or *Prostigmatid* predatory mite species;
- a food source for the mite individuals;
- and a carrier for the individuals of the mite species comprising stacked carrier elements selected from millet husks, wherein the carrier elements comprise shelters for mite individuals.

The composition is suitable for rearing a mite species. The mite species preferably is a commercially relevant species. Predatory mites and rearing preys are most preferably selected as the commercially relevant mite species.

Predatory mites may be selected from:
- *Mesostigmatid* predatory mite species such as:
   i) *Phytoseiidae* such as from:
      - the subfamily of the *Amblyseiinae,* such as from the genus *Amblyseius,* e.g. *Amblyseius andersoni, Amblyseius aerialis, Amblyseius swirskii, Amblyseius herbicolus or Amblyseius largoensis,* from the genus *Euseius* e.g. *Euseius finlandicus, Euseius hibisci, Euseius ovalis, Euseius victoriensis, Euseius stipulatus, Euseius scutalis, Euseius tularensis, Euseius addoensis, Euseius concordis, Euseius ho* or *Euseius citri,* from the genus *Neoseiuluse.g. Neoseiulus barkeri, Neoseiulus californicus, Neoseiulus cucumeris, Neoseiulus longispinosus, Neoseiulus womersleyi, Neoseiulus idaeus, Neoseiulus anonymus, Neoseiulus paspalivorus, Neoseiulus reductus* or *Neoseiulus fallacis,* from the genus *Amblydromalus e.g. Amblydromalus limonicus* from the genus *Typhlodromalus* e.g. *Typhlodromalus aripo, Typhlodromalus laila* or *Typhlodromalus peregrinus* from the genus *Typhlodromips* e.g.*Typhlodromips montdorensis,* from the genus *Phytoseiulus,* e.g. *Phytoseiulus persimilis, Phytoseiulus macropilis, Phytoseiulus longipes, Phytoseiulus fragariae;*
      - the subfamily of the *Typhlodrominae,* such as from the genus *Galendromus e.g.Galendromus occidentalis,* from the genus *Typhlodromus* e.g. *Typhlodromus pyri, Typhlodromus doreenae* or *Typhlodromus athiasae;*
   ii) *Ascidae* such as from the genus *Proctolaelaps,* such as *Proctolaelaps pygmaeus* (Muller); from the genus *Blattisocius* e.g. *Blattisocius tarsalis* (Berlese), *Blattisocius keegani* (Fox); from the genus *Lasioseius* e.g. *Lasioseius fimetorum* Karg, *Lasioseius floridensis* Berlese, *Lasioseius bispinosus* Evans, *Lasioseius dentatus* Fox, *Lasioseius scapulatus* (Kenett), *Lasioseius athiasae* Nawar & Nasr; from the genus *Arctoseius* e.g. *Arctoseius semiscissus* (Berlese); from the genus *Protogamasellus* e.g. *Protogamasellus dioscorus* Manson;
   iii) *Laelapidae* such as from the genus *Stratiolaelaps* e.g. *Stratiolaelaps scimitus*(*Womersley*) (also placed in the genus *Hypoaspis*) *; Geolaelaps* e.g. *Geolaelaps aculeifer* (Canestrini) (also placed in the genus *Hypoaspis*); *Androlaelaps* e.g. *Androlaelaps casalis casalis* (Berlese);
   iv) *Macrochelidae* such as from the genus *Macrocheles e.g.Macrocheles robustulus* (Berlese), *Macrocheles muscaedomesticae* (Scopoli), *Macrocheles matrius* (Hull);
   v) *Parasitidae* such as from the genus *Pergamasus* e.g*.Pergamasusquisquiliarum* Canestrini; *Parasitus* e.g.*Parasitusfimetorum* (Berlese), *Parasitus bituberosus* Karg;
- *Prostigmatid* mite species such as from:
   vi) *Tydeidae* such as from the genus *Homeopronematus* e.g. *Homeopronematus anconai* (Baker); from the genus *Tydeus* e.g.*Tydeus lambi* (Baker), *Tydeus caudatus* (Dugés), *Tydeus lambi* (Baker); from the genus *Pronematus* e.g. *Pronematus ubiquitous* (McGregor);
   vii) *Cheyletidae* such as from the genus *Cheyletus* e.g.*Cheylelus eruditus* (Schrank), *Cheyletus malaccensis* Oudemans;
   viii) *Cunaxidae* such as from the genus *Coleoscirus* e.g.*Coleoscirus simplex* (Ewing), from the genus *Cunaxa* e.g. *Cunaxa setirostris*(*Hermann*);
   ix) *Erythraeidae* such as from the genus *Balaustium* e.g. *Balaustium putmani* Smiley, *Balaustium medicagoense* Meyer &Ryke , *Balaustium murorum* (Hermann);
   x) *Stigmaeidae* such as from the genus *Agistemus* e.g. *Agistemus exsertus* Gonzalez; such as from the genus *Zetzellia* e.g. *Zetzelliamali*(Ewing).

The skilled person will have knowledge about the natural habitats of these and other mites suitable to be employed within the present invention and will be able to isolate them from these habitats. It may be noted that alternative and equivalent names may be in use for certain mite species. For example it is known to the skilled person that *Amblydromalus limonicus* is also known by the alternative and equivalent names *Amblyseius limonicus* and *Typhlodromalus limonicus.*

When selected as a *Phytoseiid* species, the mite species preferably is a *Phytoseiid* species selected from *Amblyseius swirskii, Amblysieus aerialis, Amblyseius andersoni, Neoseiulus barkeri, Neoseiulus californicus, Neoseiulus cucumeris, Neoseiulus fallacis, Typhlodromips montdorensis or Amblydromalus limonicus.*

Selection of an adequate food source for the *Mesostigmatid* or *Prostigmatid* predatory mite individuals is within the ambit of the knowledge of the skilled person. As the skilled person will know the suitability of the food sources will depend on the selected mite. Natural prey, rearing prey such as Astigmatid prey mites, artificial diets, eggs from *Tetranychidae,* eggs from *Lepidoptera,* such as eggs from *Ephestia* or *Sitotroga,* plant pollen, may be suitable, depending on the requirements of the mite. As the skilled person is aware of *Phytoseiulus* species require *Tetranichids,* preferably *Tetranichid* eggs, more preferably eggs from *Tetranichus urticae* as a food source.

For the *Phytoseiidae* (with the exception of *Phytoseiulus* species), *Ascidae, Laelapidae, Macrochelidae, Parasitidae, Cheyletidae, Cunaxidae, Erythraeidae* or *Stigmaeidae* rearing preys may be selected from the suborder Astigmata. The *Astigmatid* mites can be isolated from their natural habitats as described by Hughes A.M., 1977, and can be maintained and cultured as described by Parkinson, C.L. (1992) and Solomon, M.E. & Cunnington, A.M. (1963). For example suitable *Astigmatid* rearing prey species may be selected from:
i) *Carpoglyphidae* such as from the genus *Carpoglyphus* e.g. *Carpoglyphus lactis*;
ii) *Pyroglyphidae* such as from the genus *Dermatophagoides* e.g. *Dermatophagoides pteronysinus, Dermatophagoides farinae*; from the genus *Euroglyphus* e.g. *Euroglyphus longior, Euroglyphus maynei*; from the genus *Pyroglyphus* e.g. *Pyroglyphus africanus*;
iii) *Glycyphagidae* such as from the subfamily *Ctenoglyphinae,* such as from the genus *Diamesoglyphus* e.g. *Diamesoglyphus intermediusor* from the genus *Ctenoglyphus,* e.g. *Ctenoglyphus plumiger, Ctenoglyphus canestrinii, Ctenoglyphus palmifer;* the subfamily *Glycyphaginae,* such as from the genus *Blomia,* e.g. *Blomia freemani* or from the genus *Glycyphagus,* e.g. *Glycyphagus ornatus, Glycyphagus bicaudatus, Glycyphagus privatus, Glycyphagus domesticus,* or from the genus *Lepidoglyphus* e.g. *Lepidoglyphus michaeli, Lepidoglyphus fustifer, Lepidoglyphus destructor,* or from the genus *Austroglycyphagus,* e.g. *Austroglycyphagus geniculatus*; from the subfamily *Aeroglyphinae,* such as from the genus *Aeroglyphus,* e.g. *Aëroglyphus robustus;* from the subfamily *Labidophorinae,* such as from the genus *Gohieria,* e.g. *Gohieria. fusca;* or from the subfamily *Nycteriglyphinae* such as from the genus *Coproglyphus,* e.g. *Coproglyphus stammeri* or from the subfamily *Chortoglyphidae,* such as the genus *Chortoglyphus* e.g. *Chortoglyphus arcuatus* and more preferably is selected from the subfamily *Glycyphaginae,* more preferably is selected from the genus *Glycyphagus* or the genus *Lepidoglyphus* most preferably selected from *Glycyphagus domesticus or Lepidoglyphus destructor*;
iv) *Acaridae* such as from the genus *Tyrophagus* e.g. *Tyrophagus putrescentiae, Tyrophagus tropicus;* from the genus *Acarus* e.g. *Acarus siro, Acarus farris, Acarus gracilis*; from the genus *Lardoglyphus* e.g. *Lardoglyphus konoi,* from the genus *Thyreophagus,* such as *Thyreophagus entomophagus*; from the genus *Aleuroglyphus,* e.g. *Aleuroglyphus ovatus*
v) *Suidasiidae* such as from the genus *Suidasia,* such as *Suidasia nesbiti, Suidasia pontifica or Suidasia medanensis.*

A reference to the *Astigmata* is presented in Hughes (1977). Preferred Astigmatid mites may be selected from *Lepidoglyphus destructor, Carpoglyphidae* such as from the genus *Carpoglyphus* e.g. *Carpoglyphus lactis,* the genus *Thyreophagus,* such as *Thyreophagus entomophagus, Acaridae, Suidasia pontifica or Suidasia medanensis*. Or *Blomia spp.*

The composition according to the invention comprises a population of individuals of the mite species. The population preferably is a rearing population. In this description the term rearing must be understood to include the propagation and increase of a population by means of sexual reproduction. A rearing population may comprise sexually mature adults from both sexes, and/or individuals of both sexes of other life stages, e.g. eggs and/or nymphs, which can mature to sexually mature adults. Alternatively the rearing population may comprise one or more fertilized females. In essence a rearing population is capable of increasing the number of its individuals by means of sexual reproduction.

The composition of the invention furthermore comprises a carrier for the individuals of the mite species. The use of carrier materials in the rearing practice of mites such as predatory mites and rearing preys is known. The use of a carrier comprising finely divided carrier elements is popular in view of the possibility to maintain the mite culture as a three-dimensional culture. Such carriers usually comprise carrier elements, having a longest axis of about 1.0-15.0 mm, such as 3.0-9.0 mm. Bran, vermiculite, corn cob grits and sawdust are known carriers from the prior art.

In the composition of the invention the stacking of carrier elements comprises shelters for mite individuals. In general terms a shelter may be defined as a dwelling place providing refuge from disturbing external influences. The shelters of the carrier according to the invention provide such refuge to the mite individuals, in particular for juvenile life stages such as eggs, larvae and nymphs. Such shelters will protect predatory mites from disturbing influences such as motional activity, disturbance and interference by other predatory or prey mite individuals and from cannibalism by predatory mites. On the basis of the disclosure of the present invention, in combination with his common general knowledge, the skilled person will be able to understand the structural requirements for a mite shelter. Thus the skilled person will be able to design and/or select suitable carriers comprising mite shelters, in particular shelters suitable for commercially relevant mites selected from predatory mites or rearing preys.

According to an embodiment of the invention sheltering may be provided in an area where the material of the carrier element shields a mite individual, when located in this area, from its surroundings in at least 3 directions having orthogonal or reversed relations. Shielding from the surroundings should be understood as, to at least reduce, preferably to restrict and most preferably to substantially eliminate, disturbing external interactions. Such disturbing external interactions in particular are produced or brought about by other mites in the composition, such as for example movement and associated body contact with other mites. But may for example also be cannibalistic predation by individuals from the same species, in case the mite is a predatory mite. It should be understood that all predatory mites to some extend display cannibalistic behaviour. Such disturbing interactions negatively influence the population development rate because they negatively influences one or more of the oviposition rate, survival of immatures and adult longevity of the mite individuals. The intensity of these disturbing interactions will typically increase at higher population densities. However, the commercial producer of mites aim to achieve as high population densities and population development rates as possible in order to reduce the production cost as much as possible. According to an embodiment of the invention sheltering may be provided by shielding the mite individuals from the disturbing interactions. This shielding may be provided by reducing the access to the mite individuals.

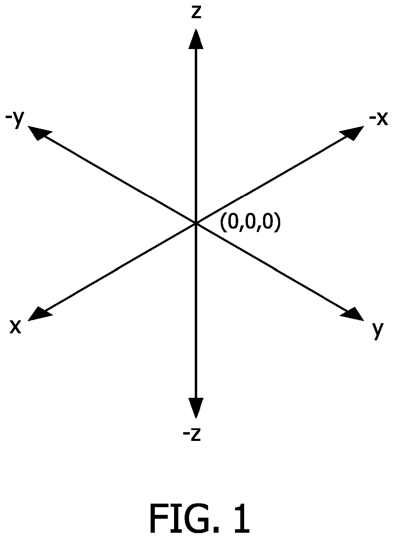

As will be understood, directions having orthogonal or reversed relations correspond to directions along the 6 axes (positive X, negative X, positive Y, negative Y, positive Z, negative Z) of an imaginary orthogonal (or Cartesian) three dimensional coordinate system in the direction out of the origin (0,0,0), where the mite individual is in the origin. These directions are either perpendicular (orthogonal) or reversed in direction. In three-dimensional space the maximal number of these directions is 6, as is depicted in figure 1.

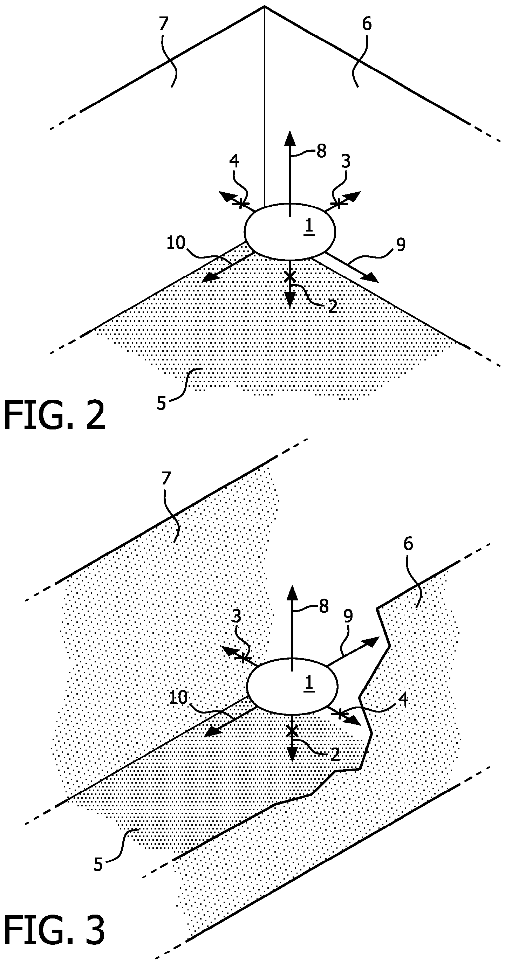

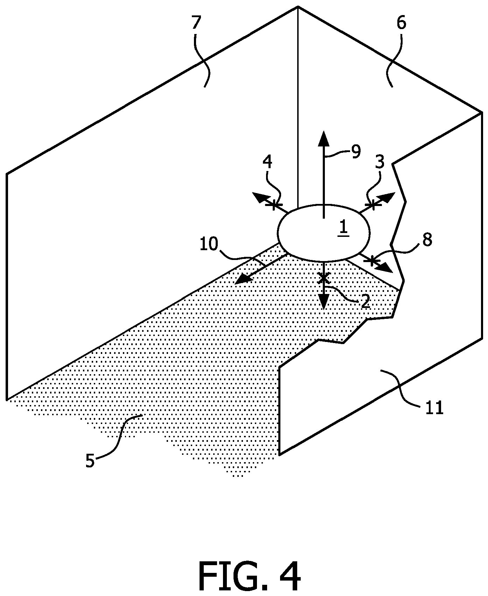

According to an embodiment of the invention the mite individual, when located in a sheltering area, is shielded from its surroundings in at least 3 such directions, preferably in at least 4 of such directions, most preferably in at least 5 of such directions, such as in 5 such directions. Shielding in 3 such directions may be provided by a structure similar to a corner formed between 3 planes such as presented in figure 2 or figure 3. Shielding in at least 4 of such directions may be provided by a structure such as a "box" open at 2 sides as presented in figure 4. Shielding in 5 directions would be provided in the situation of figure 3, where a 5^{th} horizontal plane is placed on the side wall of the 4 plane "box", such that an open cube is obtained.

In order to shield the mite individuals from external influences brought about by other mites in the composition it is preferred that the shelters are dimensioned such that the volume of the shelter is from 1-140 mm³, such as 2-120 mm³, 2-100 mm³, 2-80 mm³, 2-70 mm³, 2-60 mm³, 2-50 mm³, 2-40 mm³, 2-30 mm³, 2-25 mm³, 2-20 mm³, 2-18 mm³, 2-16 mm³, 2-14 mm³, 2-12 mm³, 2-10 mm³, 2-8 mm³, 2-6 mm³, or 2-4 mm³. This reduces the possibility that too many mite individuals are present in a shelter, which may give a disturbing effect.

It is evident that the shelters must be accessible by the mite individuals. In this respect it should be noted that areas not accessible for the mites cannot be qualified as shelters. According to certain embodiments of the invention in order to have good accessibility for mite individuals an area may have an access having an access diameter of at least 0.3- 1.2 mm, such as 0.5-1.0 mm or 0.5-0.8 mm and an access area of at least 0.25 - 1.44 mm², 0.30 - 1.20 mm², 0.30 - 1.00 mm², 0.30 - 0.80 mm², 0.30 - 0.90 mm². Depending on the maximum size of the mite species to be reared suitable carrier dimensions can be selected. For example, *Amblydromalus limonicus* (*Phytoseiidae*) are relatively small and the maximum width for females is around 0.30 mm. The same goes for *Blattisocius tarsalis* (*Ascidae*) with the same maximum width. For such mites a shelter access having an access diameter of 0.5-0.8 mm and an access area of 0.30-0.90 mm² will suffice. Females are thus able to lay eggs within the shelter, and the next stages are able to stay here or roam about. Millet chaff may provide a carrier conforming to the required dimensions. Medium sized mites such as *Cheyletus eruditus* (*Cheyletidae*) (maximum with = 0.35mm) and big mites, such as *Macrocheles muscaedomesticae* (*Macrochelidae*) (maximum width = 0.60 mm) may require a bigger husk size, such as chaff from oryza species may then be suitable.

Mite shelters may be provided by voids, such as voids formed by coves, recesses, pores, chambers, cavities, niches, pits, pockets, tubes, domes, tubs and alike structures. Such voids, preferably conforming to the dimensions presented above for the volume and/or access are suitable as mite shelters.

Shelters for the mite individuals may be present on or in individual carrier elements present in the stacking. That is to say individual carrier elements in the stacking comprise structures suitable as mite shelters. Alternatively the mite shelters may be formed between carrier elements in the stacking. That is to say in the stacking of carrier elements a plurality of carrier elements together form structures suitable as mite shelters. A "carrier element stacking" is to be understood to mean a three dimensional ordering of a multitude of carrier elements. The term "ordering" includes a random ordering.

The skilled person will know the meaning of the term chaff and will understand that chaff is the dry, scaly protective casings (husks) of the seeds of grass species (in particular cereal grains), or similar fine, dry, scaly plant material such as scaly parts of flowers, or finely chopped straw. According to the invention millet husks are used. Husks from millet have excellent external and internal dimensions which make them highly suitable as a mite rearing substrate.

Species comprised within the term millet for the present invention include: Pearl millet or Bajra (*Pennisetum glaucum*); Foxtail millet (*Setaria italica*); Proso millet, common millet, broom corn millet, hog millet or white millet (*Panicum miliaceum*); Finger millet *(Eleusine coracana)* (Also known as Ragi, Nachani or Mandwa in India), Indian barnyard millet or Sawa millet (*Echinochloafrumentacea*); Japanese barnyard millet (*Echinochloa esculenta*); Kodo millet (*Paspalum scrobiculatum*); Little millet (*Panicum sumatrense*); Guinea millet (*Brachiaria deflexa* = *Urochloa deflexa*); Browntop millet (*Urochloa ramosa* = *Brachiaria ramosa* = *Panicum ramosum*)*.* Teff (*Eragrostis tef*) and fonio (*Digitaria exilis*) are also often called millets, as more rarely are sorghum *(Sorghum* spp.) and Job's Tears (*Coix lacrima-jobi*)*.* For the present invention these species are also within the term millet.

Apart from the dimensions of the carrier elements and their structural configuration suitable to provide mite shelters, it is preferred that the carrier elements are inert in terms of biodegradation. This means that the carrier material is a poor growing substrate for microorganisms such as fungi and/or bacteria. This aids in controlling microbial growth, such as fungal growth, which is a potential problem under mite rearing conditions. Chaff and in particular the preferred chaff varieties discussed above are such poor growing substrates for fungi.

A further aspect of the invention relates to a method according to claim 7 for rearing a population of a mite species comprising:
(i) providing a composition according to invention;
(ii) allowing individuals of the mite population to feed on the food source

Methods for rearing of *mites,* such as predatory mites, wherein a population of the predator, such as a *Phytoseiid* predator, is brought in association with a food source, such as a food source comprising a population of an *Astigmatid* mite, and wherein individuals of the predator are allowed to feed on the food source are known in the art. The method according to the present invention is distinguished over the prior art methods in that in the composition according to the invention comprises carrier elements and the stacking of the carrier elements comprises shelters for mite individuals.

The technical aspects of the composition according to the invention have already been discussed above.

Yet a further aspect of the invention relates to a rearing deviced, according to claim 9, for rearing a mite species, such as a predatory mite, said system comprising a container holding the composition according to the invention. According to a preferred embodiment the container preferably comprising an exit for at least one motile life stage of the mite, more preferably an exit suitable for providing a sustained release of said at least one motile life stage.

According to another aspect the invention relates to the use of the composition of the invention or the rearing system according to the invention for controlling a crop pest.

According to this aspect the mite is selected as a predatory mite. The skilled person will know the suitability of predatory mites for controlling crop pests. For this reference may be made to Gerson et al. (2003). For example if the predatory mite is selected as a *Phytoseiid* predator. Pests that may be effectively cotrolled may be selected from white flies, such as *Trialeurodes vaporariorum* or *Bemisia tabaci*; thrips, such as *Thrips tabaci* or *Frankliniella spp.,* such as *Frankliniella occidentalis,* spider mites such as *Tetranychus urticae,* or other phytophagous mites such as *Polyphagotarsonemus.*

Crops that may benefit from treatment with the composition according to the invention may be selected from, but are not restricted to (greenhouse) vegetable crops such as peppers (*Capsicum annuum*), eggplants (*Solanum melogena*) Curcubits (*Cucurbitaceae*) such as cucumbers (*cucumis sativa*), melons (*cucumis melo*) watermelons (*Citrullus lanatus*); soft fruit (such as strawberries (*Fragaria x annanassa*), raspberries (*Rubus ideaus*)), (greenhouse) ornamental crops (such as roses, gerberas, chrysanthemums) or tree crops such as *Citrus spp.*

A further aspect of the invention relates to a method for biological pest control in a crop, according to claim 8. The method comprises providing the composition of the invention to said crop. The pest and the crop may be selected as described above.

In the method according to the invention the composition may be provided by applying an amount of said composition in the vicinity, such as on or at the basis of a number of crop plants. The composition may be provided to the crop plant simply by spreading it on the crop plant or at the basis of the crop plant as is common practice for employing predatory mite compositions for augmentative biological pest control. The amount of the composition which may be provided to each individual crop plant by way of spreading may range 20 from 1-20 ml such as 1-10 ml, preferably 2-5 ml. Alternatively the composition may be provided to the number of crop plants in the rearing system according to the invention which is suitable for releasing predatory mites in a crop. The rearing system may be placed in the vicinity, such as in or at the basis, of a number of crop. In the method for biological pest control according to the invention it may not be necessary to provide the composition to all crop plants. As commercial crops are normally densely cultivated. The predatory mites may spread from one crop plant to another. The number of crop plants which must be provided with the composition according to the invention in order to provide sufficient crop protection may depend on the specific circumstances and can be easily determined by the skilled person based on his experience in the field. Usually the number of predatory mites released per hectare is more determining. This number may range from 1000-3 million per hectare, typically 250.000 - 1 million or 250.000 - 500.000.

A further aspect of the invention, according to claim 11, relates to the use of a carrier material comprising stacked carrier elements selected from millet husks, for rearing a population of a mite species, wherein the carrier elements comprise shelters for mite individuals. As will be evident from the description above, such a carrier has certain benefits for rearing a mite such as a predatory mite and for its use as a biological control agent. Amongst others population densities may be increased relative to rearing on carriers without shelters.

Also the shelters may provide protection against mechanical stress such as the mechanical stress to which mites may be subjected during distribution in the field such as by blowing in a forced gas stream. According to an embodiment the use is therefore aimed at rearing of the mites for distribution by means of blowing.

The invention will now be further illustrated with reference to the attached figures and examples. It should be emphasized that these figures and examples are only illustrative and by no means restrict the scope of the invention as defined in the embodiments.
Figure 1 presents an three dimensional orthogonal (Cartesian) coordinate system. Along the axes X,Y,Z six directions out of the origin (0,0,0) may be defined (along positive X, along negative X, along positive Y, along negative Y, along positive Z, along negative Z). These directions are either perpendicular (orthogonal) or reversed in direction.
Figure 2 presents a schematic overview of a shelter wherein a mite individual (1) is shielded from interaction with its surrounding in three directions indicated by arrows (2), (3), (4). The sheltering is provided by a floor plane (5), a first side plane (6) and a second side plane (7). Interacting influences may still come from the surroundings from directions indicated by arrows (8), (9), (10).
Figure 3 presents a schematic overview of an alternative shelter wherein a mite individual (1) is shielded from interaction with its surrounding in three directions indicated by arrows (2), (3), (4). The sheltering is provided by a floor plane (5), a first side plane (6) and a second side plane (7). Interacting influences may still come from the surroundings from directions indicated by arrows (8), (9), (10).
Figure 4 presents a schematic overview of a shelter wherein a mite individual (1) is shielded from interaction with its surrounding in four directions indicated by arrows (2), (3), (4), (8). The sheltering is provided by a floor plane (5), a first side plane (6), a second side plane (7) and a third side plane (11). Interacting influences may still come from the surroundings from directions indicated by arrows (9), (10). It will be clear that the mite individual may be further shielded from interactions from the surroundings if a covering plane is located on the side planes (6), (7), (11). In addition, shielding from the surroundings may be further enhanced if a further side plane would be placed perpendicular to side plane (7). In this way the mite individual (1) would also be shielded from the surrounding in the direction indicated by arrow (10).

It should be understood that while all schematic representations of figures 1-4 are presented in rectangular geometry, similar shielding effects may be provided by non rectangular structures such as coves, recesses, pores, chambers, cavities, niches, pits, pockets, tubes, domes, tubs and alike structures.

### EXAMPLE I

### Setup

Two species of predatory mites, A. *swirskii* and A. *limonicus,* were tested with respect to their preference for different carrier types. Mature females were collected approximately 10 days after the start of rearing from the egg stage. The 3 offered carriers were millet chaff, a carrier according to the invention, wheat bran, standard carrier and vermicullite (fine grain, all particles < 2 mm), also a standard carrier. All carriers were simultaneously offered in a moist form (15 ml water/100g added). Of each carrier 2 portions were placed opposite one another on a fixed distance from the release point (4 cm). The tested substrates were all offered in the same volume of 0.5 cc (divided in 2 portions per arena). At the start of the test, 10 females and 2 males of each species were placed in the middle of each plastic choice arena (Ø = 12 cm). The arena was placed on moist cotton wool to offer water for the predatory mites and to prevent escape. *Typha* pollen was placed as a food source at the release point. The number of replicates was 3 and each subsequent arena was orientated with another substrate at top position (12 o'clock).

The test was performed in a climate room with conditions of 25°C, 75% RH and 16:8 (L:D) light regime and the RH on the arena was around 85%. After 2 days the number of predator eggs per substrate and the number of adults present were counted(male individuals were excluded from the statistics). For this all carrier particles were scrutinized individually and also checked 2 days later after extra food was added. The results per substrate per species were statistically analysed using the Chi-square Goodness of Fit Test (one variable).

### Results

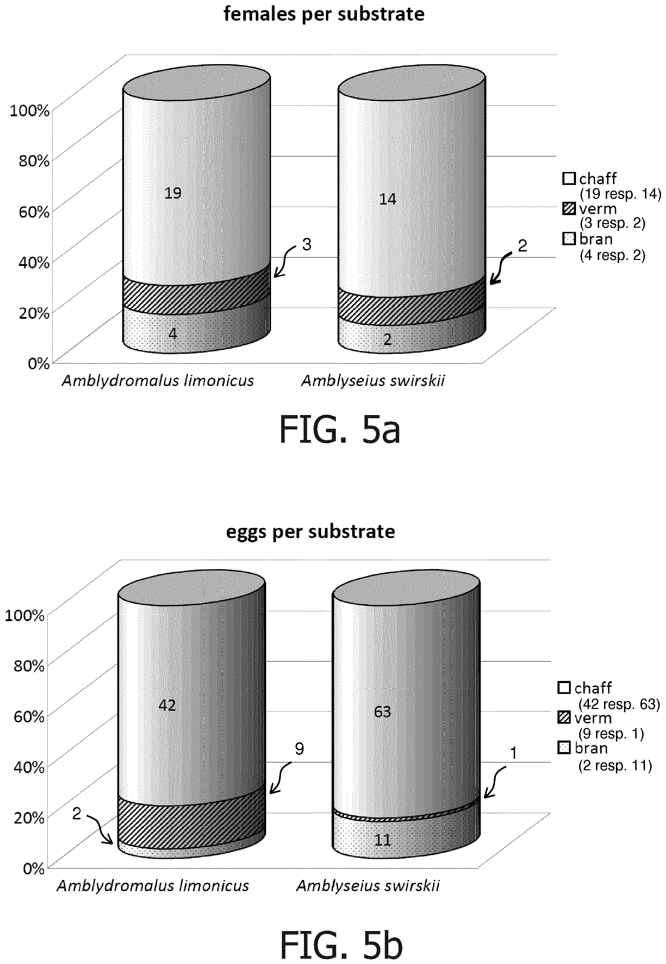

The total number of females found in each substrate (after 3 replicates) is presented in figure 5 (panel A). Of all start-up females (30) a large fraction of individuals was retrieved from the substrates, i.e. 87% (26 individuals) of all A. *limonicus* and 60% (18 individuals) of all A. *swirskii.* Thus even though the material was clearly separated from the food source, the majority of female mites were found in this carrier. Both tests showed a significant difference between carrier materials (p=0.000).

The total number of eggs (and hatchlings) found in each carrier (after 3 replicates) is shown in panel B of figure 5. It is clear that the occurrence of female mites correlates with the number of eggs laid on the carriers. Both tests showed a significant difference between carrier materials (p=0.000).

The results indicate that carrier materials providing mite shelters, as represented by the millet chaff in this experiment, are a highly preferred for mite species, such as predatory mite species, in particular *Phytoseiid* species.

### EXAMPLE II

### Setup

Thick layers of medium were prepared to simulate a mass-rearing unit. Either bran or millet chaff (both moistened) were used as the carrier material. Bran is the standard carrier used in commercial mite rearing. Chaff is a representative for carriers according to the invention with mite shelters. Two food types (A and B), both comprising *Carpoglyphus lactis* in frozen form were used. In a start-up rearing the predator mite, A. *limonicus,* was reared for >2 generations on the test medium in thin layers. The subsequent rearing was performed in layers of 6-7 cm high in ventilated boxes (LxWxH=15x15x8cm) during 2 weeks. Sampling, feeding and mixing was done twice a week. The test was performed in duplo at 21°C and 93% RH. Each week the number of live predator and prey mites were counted from the sample.

### Results

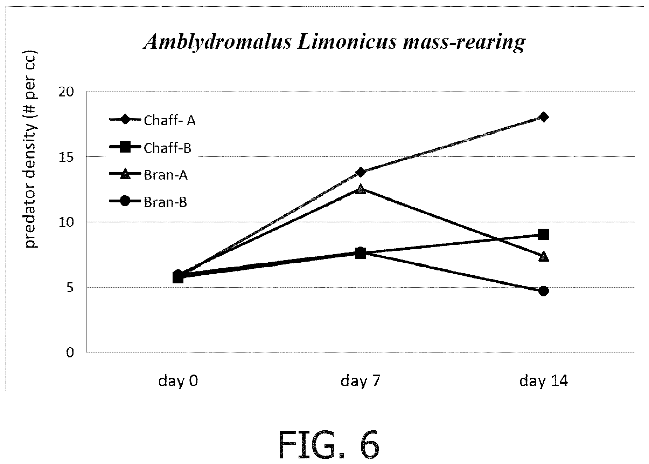

The results are presented in figure 6. The predator densities in the chaff rearings are increasing in the first and second week, on both food types. In the bran mixes, the rearings are keeping up in the first week, but collapse in the second week. The decrease of predator numbers is followed by an increase of prey mite numbers and this makes continuity of these rearing mixes troublesome. The test shows a net result that is positive for the chaff carrier as compared to the standard bran carrier.

### References

Solomon, M.E. and Cunnington, A.M., 1963, Rearing acaroid mites, Agricultural Research Council, Pest Infestation Laboratory, Slough, England, pp 399-403.
Parkinson, C.L., 1992, "Culturing free-living astigmatid mites." Arachnida: Proceedings of a one day symposium on spiders and their allies held on Saturday 21st November 1987 at the Zoological Society of London, eds. Cooper,J.E., Pearce-Kelly,P, Williams,D.L., p. 62-70.
Hughes, A.M., 1977, The mites of stored food and houses. Ministry of Agriculture, Fisheries and Food, Technical Bulletin No. 9: 400 pp
De Moraes, G.J., McMurtry, J.A., Denmark, H.A. & Campos, C.B., 2004. A revised catalog of the mite family Phytoseiidae. Magnolia Press Auckland New Zealand 494 pp.

## Claims

1. Mite composition comprising:
- a population of individuals (1) of a mite species, preferably a mite species selected from *Mesostigmatid* mite species or *Prostigmatid* mite species;
- a food source for the mite individuals;
- and a carrier for the individuals of the mite species comprising stacked carrier elements selected from millet husks
wherein the carrier elements comprise shelters for mite individuals.

2. Composition according to claim 1, wherein the shelters comprise areas where the material of the carrier element shields a mite individual, when located in this area, from its surroundings in at least 3 directions having orthogonal or reversed relations, preferably in at least 4 of such directions, most preferably in at least 5 of such directions.

3. Composition according to any of the claims 1-2, wherein the shelters comprise voids, such as voids formed by coves, recesses, pores, chambers, cavities, niches, pits, pockets, tubes and alike structures.

4. Composition according to any of the claims 1-3, wherein the millet husks are from millet selected from Pearl millet or Bajra (*Pennisetum glaucum*), Foxtail millet (*Setaria italica*), Proso millet, common millet, broom corn millet, hog millet or white millet (*Panicum miliaceum*)*,* Finger millet *(Eleusine coracana),* Indian barnyard millet or Sawa millet (*Echinochloafrumentacea*), Japanese barnyard millet (*Echinochloa esculenta*), Kodo millet (*Paspalum scrobiculatum*), Little millet (*Panicum sumatrense*), Guinea millet (*Brachiaria deflexa* = *Urochloa deflexa*), Browntop millet (*Urochloa ramosa* = *Brachiaria ramosa* = *Panicum ramosum*), Teff (*Eragrostis tef*), fonio (*Digitaria exilis*), sorghum *(Sorghum* spp.) or Job's Tears (*Coix lacrima-jobi*)*.*

5. Composition according to any of the previous claims, wherein the mite species is selected from:
- Mesostigmatid mite species such as selected from:
i) *Phytoseiidae* such as from:
- the subfamily of the *Amblyseiinae,* such as from the genus *Amblyseius,* e.g. *Amblyseius andersoni, Amblyseius aerialis, Amblyseius swirskii, Amblyseius herbicolus or Amblyseius largoensis,* from the genus *Euseius* e.g. *Euseius finlandicus, Euseius hibisci, Euseius ovalis, Euseius victoriensis, Euseius stipulatus, Euseius scutalis, Euseius tularensis, Euseius addoensis, Euseius concordis, Euseius ho* or *Euseius citri,* from the genus *Neoseiuluse*.g. *Neoseiulus barkeri, Neoseiulus californicus, Neoseiulus cucumeris, Neoseiulus longispinosus, Neoseiulus womersleyi, Neoseiulus idaeus, Neoseiulus anonymus, Neoseiulus paspalivorus, Neoseiulus reductus* or *Neoseiulus fallacis,* from the genus *Amblydromalus e.g. Amblydromalus limonicus* from the genus *Typhlodromalus* e.g. *Typhlodromalus aripo, Typhlodromalus laila* or *Typhlodromalus peregrinus* from the genus *Typhlodromips* e.g.*Typhlodromips montdorensis,* from the genus *Phytoseiulus,* e.g. *Phytoseiulus persimilis, Phytoseiulus macropilis, Phytoseiulus longipes, Phytoseiulus fragariae*;
- the subfamily of the *Typhlodrominae,* such as from the genus *Galendromus e.g.Galendromus occidentalis,* from the genus *Typhlodromus* e.g. *Typhlodromus pyri, Typhlodromus doreenae* or *Typhlodromus athiasae;*
ii) *Ascidae* such as from the genus *Proctolaelaps,* such as *Proctolaelaps pygmaeus* (Muller); from the genus *Blattisocius* e.g. *Blattisocius tarsalis* (Berlese), *Blattisocius keegani* (Fox); from the genus *Lasioseius* e.g. *Lasioseius fimetorum* Karg, *Lasioseius floridensis* Berlese, *Lasioseius bispinosus* Evans, *Lasioseius dentatus* Fox, *Lasioseius scapulatus* (Kenett), *Lasioseius athiasae* Nawar & Nasr; from the genus *Arctoseius* e.g. *Arctoseius semiscissus* (Berlese); from the genus *Protogamasellus* e.g. *Protogamasellus dioscorus* Manson;
iii) *Laelapidae* such as from the genus *Stratiolaelaps* e.g. *Stratiolaelaps scimitus(Womersley)* (also placed in the genus *Hypoaspis*) ; *Geolaelaps* e.g. *Geolaelaps aculeifer* (Canestrini) (also placed in the genus *Hypoaspis*); *Androlaelaps* e.g. *Androlaelaps casalis casalis* (Berlese);
iv) *Macrochelidae* such as from the genus *Macrocheles e.g.Macrocheles robustulus* (Berlese), *Macrocheles muscaedomesticae* (Scopoli), *Macrocheles matrius* (Hull);
v) *Parasitidae* such as from the genus *Pergamasus* e.g*.Pergamasusquisquiliarum* Canestrini; *Parasitus e.g.Parasitusfimetorum* (Berlese), *Parasitus bituberosus* Karg;
- *Prostigmatid* mite species such as from:
vi) *Tydeidae* such as from the genus *Homeopronematus* e.g. *Homeopronematus anconai* (Baker); from the genus *Tydeus* e.g.*Tydeus lambi* (Baker), *Tydeus caudatus* (Dugés), *Tydeus lambi* (Baker); from the genus *Pronematus* e.g. *Pronematus ubiquitous* (McGregor);
vii) *Cheyletidae* such as from the genus *Cheyletus* e.g.*Cheyletus eruditus* (Schrank), *Cheyletus malaccensis* Oudemans;
viii) *Cunaxidae* such as from the genus *Coleoscirus* e.g.*Coleoscirus simplex* (Ewing), from the genus *Cunaxa* e.g. *Cunaxa setirostris*(Hermann);
ix) *Erythraeidae* such as from the genus *Balaustium* e.g. *Balaustium putmani* Smiley, *Balaustium medicagoense* Meyer &Ryke , *Balaustium murorum* (Hermann);
x) *Stigmaeidae* such as from the genus *Agistemus* e.g. *Agistemus exsertus* Gonzalez; such as from the genus *Zetzellia* e.g. *Zetzelliamali*(Ewing).

6. Composition according to any of the claims 1-5 wherein the mite species is a *Phytoseiid* species, preferably a *Phytoseiid* species selected from *Amblyseius swirskii, Amblysieus aerialis, Amblyseius andersoni, Neoseiulus barkeri, Neoseiulus californicus, Neoseiulus cucumeris, Neoseiulus fallacis, Typhlodromips montdorensis or Amblydromalus limonicus.*

7. Method for rearing a population of a mite species comprising:
(i) providing a composition according to claims 1-6;
(ii) allowing individuals of the mite population to feed on the food source.

8. Method for biological pest control in a crop comprising, providing to said crop a composition according to any of the claims 1-6, wherein the mite species is selected as a predatory mite species, such as a predatory mite species selected from:
- Mesostigmatid mite species such as selected from:
i) *Phytoseiidae* such as from:
- the subfamily of the *Amblyseiinae,* such as from the genus *Amblyseius,* e.g. *Amblyseius andersoni, Amblyseius aerialis, Amblyseius swirskii, Amblyseius herbicolus or Amblyseius largoensis,* from the genus *Euseius* e.g. *Euseius finlandicus, Euseius hibisci, Euseius ovalis, Euseius victoriensis, Euseius stipulatus, Euseius scutalis, Euseius tularensis, Euseius addoensis, Euseius concordis, Euseius ho* or *Euseius citri,* from the genus *Neoseiuluse.g. Neoseiulus barkeri, Neoseiulus californicus, Neoseiulus cucumeris, Neoseiulus longispinosus, Neoseiulus womersleyi, Neoseiulus idaeus, Neoseiulus anonymus, Neoseiulus paspalivorus, Neoseiulus reductus* or *Neoseiulus fallacis,* from the genus *Amblydromalus e.g. Amblydromalus limonicus* from the genus *Typhlodromalus* e.g. *Typhlodromalus aripo, Typhlodromalus laila* or *Typhlodromalus peregrinus* from the genus *Typhlodromips* e.g.*Typhlodromips montdorensis,* from the genus *Phytoseiulus,* e.g. *Phytoseiulus persimilis, Phytoseiulus macropilis, Phytoseiulus longipes, Phytoseiulus fragariae;*
- the subfamily of the *Typhlodrominae,* such as from the genus *Galendromus e.g.Galendromus occidentalis,* from the genus *Typhlodromus* e.g. *Typhlodromus pyri, Typhlodromus doreenae* or *Typhlodromus athiasae;*
ii) *Ascidae* such as from the genus *Proctolaelaps,* such as *Proctolaelaps pygmaeus* (Muller); from the genus *Blattisocius* e.g. *Blattisocius tarsalis* (Berlese), *Blattisocius keegani* (Fox); from the genus *Lasioseius* e.g. *Lasioseius fimetorum* Karg, *Lasioseius floridensis* Berlese, *Lasioseius bispinosus* Evans, *Lasioseius dentatus* Fox, *Lasioseius scapulatus* (Kenett), *Lasioseius athiasae* Nawar & Nasr; from the genus *Arctoseius* e.g. *Arctoseius semiscissus* (Berlese); from the genus *Protogamasellus* e.g. *Protogamasellus dioscorus* Manson;
iii) *Laelapidae* such as from the genus *Stratiolaelaps* e.g. *Stratiolaelaps scimitus(Womersley)* (also placed in the genus *Hypoaspis*) *; Geolaelaps* e.g. *Geolaelaps aculeifer* (Canestrini) (also placed in the genus *Hypoaspis*); *Androlaelaps* e.g. *Androlaelaps casalis casalis* (Berlese);
iv) *Macrochelidae* such as from the genus *Macrocheles e.g.Macrocheles robustulus* (Berlese), *Macrocheles muscaedomesticae* (Scopoli), *Macrocheles matrius* (Hull);
v) *Parasitidae* such as from the genus *Pergamasus* e.g*.Pergamasusquisquiliarum* Canestrini; *Parasitus* e.g.*Parasitusfimetorum* (Berlese), *Parasitus bituberosus* Karg;
- *Prostigmatid* mite species such as from:
vi) *Tydeidae* such as from the genus *Homeopronematus* e.g. *Homeopronematus anconai* (Baker); from the genus *Tydeus* e.g.*Tydeus lambi* (Baker), *Tydeus caudatus* (Dugés), *Tydeus lambi* (Baker); from the genus *Pronematus* e.g. *Pronematus ubiquitous* (McGregor);
vii) *Cheyletidae* such as from the genus *Cheyletus* e.g.*Cheylelus eruditus* (Schrank), *Cheyletus malaccensis* Oudemans;
viii) *Cunaxidae* such as from the genus *Coleoscirus* e.g.*Coleoscirus simplex* (Ewing), from the genus *Cunaxa* e.g. *Cunaxa setirostris*(*Hermann*);
ix) *Erythraeidae* such as from the genus *Balaustium* e.g. *Balaustium putmani* Smiley, *Balaustium medicagoense* Meyer &Ryke , *Balaustium murorum* (Hermann);
x) *Stigmaeidae* such as from the genus *Agistemus* e.g. *Agistemus exsertus* Gonzalez; such as from the genus *Zetzellia* e.g. *Zetzelliamali*(Ewing).

9. Rearing device for rearing a mite species, said device comprising a container holding the composition according to any of the claims 1-6, preferably a container comprising an exit for at least one motile life stage of the mite species, more preferably an exit suitable for providing a sustained release of said at least one motile life stage.

10. Use for crop protection of a composition according to any of the claims 1-6, wherein the mite species is selected as a predatory mite species, such as a predatory mite species selected from:
- Mesostigmatid mite species such as selected from:
i) *Phytoseiidae* such as from:
- the subfamily of the *Amblyseiinae,* such as from the genus *Amblyseius,* e.g. *Amblyseius andersoni, Amblyseius aerialis, Amblyseius swirskii, Amblyseius herbicolus or Amblyseius largoensis,* from the genus *Euseius* e.g. *Euseius finlandicus, Euseius hibisci, Euseius ovalis, Euseius victoriensis, Euseius stipulatus, Euseius scutalis, Euseius tularensis, Euseius addoensis, Euseius concordis, Euseius ho* or *Euseius citri,* from the genus *Neoseiuluse.g. Neoseiulus barkeri, Neoseiulus californicus, Neoseiulus cucumeris, Neoseiulus longispinosus, Neoseiulus womersleyi, Neoseiulus idaeus, Neoseiulus anonymus, Neoseiulus paspalivorus, Neoseiulus reductus* or *Neoseiulus fallacis,* from the genus *Amblydromalus e.g. Amblydromalus limonicus* from the genus *Typhlodromalus* e.g. *Typhlodromalus aripo, Typhlodromalus laila* or *Typhlodromalus peregrinus* from the genus *Typhlodromips e.g.Typhlodromips montdorensis,* from the genus *Phytoseiulus,* e.g. *Phytoseiulus persimilis, Phytoseiulus macropilis, Phytoseiulus longipes, Phytoseiulus fragariae;*
- the subfamily of the *Typhlodrominae,* such as from the genus *Galendromus e.g.Galendromus occidentalis,* from the genus *Typhlodromus* e.g. *Typhlodromus pyri, Typhlodromus doreenae* or *Typhlodromus athiasae;*
ii) *Ascidae* such as from the genus *Proctolaelaps,* such as *Proctolaelaps pygmaeus* (Muller); from the genus *Blattisocius* e.g. *Blattisocius tarsalis* (Berlese), *Blattisocius keegani* (Fox); from the genus *Lasioseius* e.g. *Lasioseius fimetorum* Karg, *Lasioseius floridensis* Berlese, *Lasioseius bispinosus* Evans, *Lasioseius dentatus* Fox, *Lasioseius scapulatus* (Kenett), *Lasioseius athiasae* Nawar & Nasr; from the genus *Arctoseius* e.g. *Arctoseius semiscissus* (Berlese); from the genus *Protogamasellus* e.g. *Protogamasellus dioscorus* Manson;
iii) *Laelapidae* such as from the genus *Stratiolaelaps* e.g. *Stratiolaelaps scimitus(Womersley)* (also placed in the genus *Hypoaspis*) *; Geolaelaps* e.g. *Geolaelaps aculeifer* (Canestrini) (also placed in the genus *Hypoaspis*); *Androlaelaps* e.g. *Androlaelaps casalis casalis* (Berlese);
iv) *Macrochelidae* such as from the genus *Macrocheles e.g.Macrocheles robustulus* (Berlese), *Macrocheles muscaedomesticae* (Scopoli), *Macrocheles matrius* (Hull);
v) *Parasitidae* such as from the genus *Pergamasus* e.g*.Pergamasusquisquiliarum* Canestrini; *Parasitus* e.g.*Parasitusfimetorum* (Berlese), *Parasitus bituberosus* Karg;
- *Prostigmatid* mite species such as from:
vi) *Tydeidae* such as from the genus *Homeopronematus* e.g. *Homeopronematus anconai* (Baker); from the genus *Tydeus* e.g.*Tydeus lambi* (Baker), *Tydeus caudatus* (Dugés), *Tydeus lambi* (Baker); from the genus *Pronematus* e.g. *Pronematus ubiquitous* (McGregor);
vii) *Cheyletidae* such as from the genus *Cheyletus* e.g.*Cheyletus eruditus* (Schrank), *Cheyletus malaccensis* Oudemans;
viii) *Cunaxidae* such as from the genus *Coleoscirus* e.g.*Coleoscirus simplex* (Ewing), from the genus *Cunaxa* e.g. *Cunaxa setirostris*(Hermann);
ix) *Erythraeidae* such as from the genus *Balaustium* e.g. *Balaustium putmani* Smiley, *Balaustium medicagoense* Meyer *&*Ryke, *Balaustium murorum* (Hermann);
x) *Stigmaeidae* such as from the genus *Agistemus* e.g. *Agistemus exsertus* Gonzalez; such as from the genus *Zetzellia* e.g. *Zetzelliamali*(Ewing).

11. Use of a carrier material comprising stacked carrier elements selected from millet husks, , for rearing a population of a mite species, preferably a mite species selected from *Mesostigmatid* mite species or *Prostigmatid* mite species, wherein the carrier elements comprise shelters for mite individuals.

12. Use according to claim 11, wherein the shelters comprise areas where the carrier material of the carrier element shields a mite individual, when located in this area, from its surroundings in at least 3 directions having orthogonal or reversed relations, preferably in at least 4 of such directions, most preferably in at least 5 of such directions.

13. Use according to any of the claims 11-12, wherein the shelters comprise voids, such as voids formed by coves, recesses, pores, chambers, cavities, niches, pits, pockets, tubes and alike structures.

14. Use according to any of the claims 11-13, wherein the millet husks are from millet selected from Pearl millet or Bajra (*Pennisetum glaucum*), Foxtail millet (*Setaria italica*), Proso millet, common millet, broom corn millet, hog millet or white millet (*Panicum miliaceum*), Finger millet *(Eleusine coracana),* Indian barnyard millet or Sawa millet (*Echinochloafrumentacea*), Japanese barnyard millet (*Echinochloa esculenta*), Kodo millet (*Paspalum scrobiculatum*), Little millet (*Panicum sumatrense*), Guinea millet (*Brachiaria deflexa* = *Urochloa deflexa*), Browntop millet (*Urochloa ramosa* = *Brachiaria ramosa* = *Panicum ramosum*), Teff (*Eragrostis tef*), fonio (*Digitaria exilis*), sorghum *(Sorghum* spp.) or Job's Tears (*Coix lacrima-jobi*)*.*

15. Use according to claims 11-14, wherein the mite species is a predatory mite species, such as a predatory mite species selected from:
- Mesostigmatid mite species such as selected from:
i) *Phytoseiidae* such as from:
- the subfamily of the *Amblyseiinae,* such as from the genus *Amblyseius,* e.g. *Amblyseius andersoni, Amblyseius aerialis, Amblyseius swirskii, Amblyseius herbicolus or Amblyseius largoensis,* from the genus *Euseius* e.g. *Euseius finlandicus, Euseius hibisci, Euseius ovalis, Euseius victoriensis, Euseius stipulatus, Euseius scutalis, Euseius tularensis, Euseius addoensis, Euseius concordis, Euseius ho* or *Euseius citri,* from the genus *Neoseiuluse.g. Neoseiulus barkeri, Neoseiulus californicus, Neoseiulus cucumeris, Neoseiulus longispinosus, Neoseiulus womersleyi, Neoseiulus idaeus, Neoseiulus anonymus, Neoseiulus paspalivorus, Neoseiulus reductus* or *Neoseiulus fallacis,* from the genus *Amblydromalus e.g. Amblydromalus limonicus* from the genus *Typhlodromalus* e.g. *Typhlodromalus aripo, Typhlodromalus laila* or *Typhlodromalus peregrinus* from the genus *Typhlodromips* e.g.*Typhlodromips montdorensis,* from the genus *Phytoseiulus,* e.g. *Phytoseiulus persimilis, Phytoseiulus macropilis, Phytoseiulus longipes, Phytoseiulus fragariae*;
- the subfamily of the *Typhlodrominae,* such as from the genus *Galendromus e.g.Galendromus occidentalis,* from the genus *Typhlodromus* e.g. *Typhlodromus pyri, Typhlodromus doreenae* or *Typhlodromus athiasae;*
ii) *Ascidae* such as from the genus *Proctolaelaps,* such as *Proctolaelaps pygmaeus* (Muller); from the genus *Blattisocius* e.g. *Blattisocius tarsalis* (Berlese), *Blattisocius keegani* (Fox); from the genus *Lasioseius* e.g. *Lasioseius fimetorum* Karg, *Lasioseius floridensis* Berlese, *Lasioseius bispinosus* Evans, *Lasioseius dentatus* Fox, *Lasioseius scapulatus* (Kenett), *Lasioseius athiasae* Nawar & Nasr; from the genus *Arctoseius* e.g. *Arctoseius semiscissus* (Berlese); from the genus *Protogamasellus* e.g. *Protogamasellus dioscorus* Manson;
iii) *Laelapidae* such as from the genus *Stratiolaelaps* e.g. *Stratiolaelaps scimitus(Womersley)* (also placed in the genus *Hypoaspis*) *; Geolaelaps* e.g. *Geolaelaps aculeifer* (Canestrini) (also placed in the genus *Hypoaspis*); *Androlaelaps* e.g. *Androlaelaps casalis casalis* (Berlese);
iv) *Macrochelidae* such as from the genus *Macrocheles e.g.Macrocheles robustulus* (Berlese), *Macrocheles muscaedomesticae* (Scopoli), *Macrocheles matrius* (Hull);
v) *Parasitidae* such as from the genus *Pergamasus* e.g*.Pergamasusquisquiliarum* Canestrini; *Parasitus* e.g.*Parasitusfimetorum* (Berlese), *Parasitus bituberosus* Karg;
- *Prostigmatid* mite species such as from:
vi) *Tydeidae* such as from the genus *Homeopronematus* e.g. *Homeopronematus anconai* (Baker); from the genus *Tydeus e.g.Tydeus lambi* (Baker), *Tydeus caudatus* (Dugés), *Tydeus lambi* (Baker); from the genus *Pronematus* e.g. *Pronematus ubiquitous* (McGregor);
vii) *Cheyletidae* such as from the genus *Cheyletus* e.g.*Cheyletus eruditus* (Schrank), *Cheyletus malaccensis* Oudemans;
viii) *Cunaxidae* such as from the genus *Coleoscirus* e.g.*Coleoscirus simplex* (Ewing), from the genus *Cunaxa* e.g. *Cunaxa setirostris*(*Hermann*);
ix) *Erythraeidae* such as from the genus *Balaustium* e.g. *Balaustium putmani* Smiley, *Balaustium medicagoense* Meyer &Ryke , *Balaustium murorum* (Hermann);
x) *Stigmaeidae* such as from the genus *Agistemus* e.g. *Agistemus exsertus* Gonzalez; such as from the genus *Zetzellia* e.g. *Zetzelliamali*(Ewing).

## Patentansprüche

1. Milbenzusammensetzung, umfassend:
- eine Population von Individuen (1) einer Milbenart, vorzugsweise einer Milbenart ausgewählt aus *Mesostigmatiden-*Milbenarten oder *Prostigmatiden*-Milbenarten;
- eine Nahrungsquelle für die Milbenindividuen;
- und einen Träger für die Individuen der Milbenart, der gestapelte Trägerelemente umfasst, ausgewählt aus Hirsespelzen,
wobei die Trägerelemente Schutzvorrichtungen für Milbenindividuen umfassen.

2. Zusammensetzung nach Anspruch 1, wobei die Schutzvorrichtungen Bereiche umfassen, in denen das Material des Trägerelements ein Milbenindividuum, wenn es sich in diesem Bereich befindet, von seiner Umgebung in mindestens 3 Richtungen mit orthogonalen oder umgekehrten Beziehungen, vorzugsweise in mindestens 4 solcher Richtungen, am meisten bevorzugt in mindestens 5 solcher Richtungen, schützt.

3. Zusammensetzung nach einem der Ansprüche 1 bis 2, wobei die Schutzvorrichtungen Hohlräume umfassen, wie etwa Hohlräume, die durch Buchten, Vertiefungen, Poren, Kammern, Aussparungen, Nischen, Gruben, Taschen, Rohre und ähnliche Strukturen gebildet werden.

4. Zusammensetzung nach einem der Ansprüche 1 bis 3, wobei die Hirsespelzen von Hirse stammen, die ausgewählt ist aus Perlhirse oder Bajra (*Pennisetum glaucum*), Kolbenhirse (*Setaria italica*), Proso-Hirse, gemeiner Hirse, Rispenhirse, Schweinehirse oder weißer Hirse *(Panicum miliaceum),* Fingerhirse *(Eleusine coracana),* indischer Stallhirse oder Sawahirse (*Echinochloa frumentacea*), Hühnerhirse (*Echinochloa esculenta*), Kodohirse (*Paspalum scrobiculatum*), Zwerghirse (*Panicum sumatrense*), Guineahirse (*Brachiaria deflexa* = *Urochloa deflexa*), Braunhirse (*Urochloa ramosa* = *Brachiaria ramosa* = *Panicum ramosum*), Teff (*Eragrostis tef*), Foniohirse (*Digitaria exilis*), Sorghum *(Sorghum spp.*) oder Hiobstränengras (*Coix lacrima-jobi*)*.*

5. Zusammensetzung nach einem der vorstehenden Ansprüche, wobei die Milbenart ausgewählt ist aus:
- Mesostigmatiden-Milbenarten, wie etwa ausgewählt aus
*i) Phytoseiidae,* wie etwa aus:
- der Unterfamilie der *Amblyseiinae,* wie etwa aus der Gattung *Amblyseius,* z. B. *Amblyseius andersoni, Amblyseius aerialis, Amblyseius swirskii, Amblyseius herbicolus* oder *Amblyseius largoensis,* aus der Gattung *Euseius,* z. B. *Euseius finlandicus, Euseius hibisci, Euseius ovalis, Euseius victoriensis, Euseius stipulatus, Euseius scutalis, Euseius tularensis, Euseius addoensis, Euseius concordis, Euseius ho* oder *Euseius citri,* aus der Gattung *Neoseiulus,* z. B. *Neoseiulus barkeri, Neoseiulus californicus, Neoseiulus cucumeris, Neoseiulus longispinosus, Neoseiulus womersleyi, Neoseiulus idaeus, Neoseiulus anonymus, Neoseiulus paspalivorus, Neoseiulus reductus* oder *Neoseiulus fallacis,* aus der Gattung *Amblydromalus*, z. B. *Amblydromalus limonicus* aus der Gattung *Typhlodromalus*, z. B. *Typhlodromalus aripo, Typhlodromalus laila* oder *Typhlodromalus peregrinus* aus der Gattung *Typhlodromips*, z. B. *Typhlodromips montdorensis,* aus der Gattung *Phytoseiulus*, z. B. *Phytoseiulus persimilis, Phytoseiulus macropilis, Phytoseiulus longipes, Phytoseiulus fragariae*;
- der Unterfamilie der *Typhlodrominae,* wie etwa aus der Gattung *Galendromus,* z. B. *Galendromus occidentalis,* aus der Gattung *Typhlodromus*, z. B. *Typhlodromus pyri, Typhlodromus doreenae* oder *Typhlodromus athiasae;*
*ii) Ascidae* wie etwa aus der Gattung *Proctolaelaps,* wie etwa *Proctolaelaps pygmaeus* (Muller); aus der Gattung *Blattisocius*, z. B. *Blattisocius tarsalis* (Berlese), *Blattisocius keegani* (Fox); aus der Gattung *Lasioseius*, z. B. *Lasioseius fimetorum* Karg, *Lasioseius floridensis* Berlese, *Lasioseius bispinosus* Evans, *Lasioseius dentatus* Fox, *Lasioseius scapulatus* (Kenett), *Lasioseius athiasae* Nawar & Nasr; aus der Gattung *Arctoseius*, z. B. *Arctoseius semiscissus* (Berlese); aus der Gattung *Protogamasellus*, z. B. *Protogamasellus dioscorus* Manson;
iii) *Laelapidae* wie etwa aus der Gattung *Stratiolaelaps*, z. B. *Stratiolaelaps scimitus* (Womersley) (auch in die Gattung *Hypoaspis* eingeordnet); *Geolaelaps*, z. B. *Geolaelaps aculeifer* (Canestrini) (auch in die Gattung *Hypoaspis* eingeordnet); *Androlaelaps*, z. B. *Androlaelaps casalis casalis* (Berlese);
iv) *Macrochelidae* wie etwa aus der Gattung *Macrocheies*, z. B. *Macrocheies robustulus* (Berlese), *Macrocheies muscaedomesticae* (Scopoli), *Macrocheies matrius* (Hull);
v) *Parasitidae* wie etwa aus der Gattung *Pergamasus,* z. B. *Pergamasus quisquiliarum* Canestrini; *Parasitus,* z. B. *Parasitus fimetorum* (Berlese), *Parasitus bituberosus* Karg;
- *Prostigmatiden-Milbenarten* wie etwa aus:
vi) *Tydeidae* wie etwa aus der Gattung *Homeopronematus*, z. B. *Homeopronematus anconai* (Baker); aus der Gattung *Tydeus*, z. B. *Tydeus lambi* (Baker), *Tydeus caudatus* (Duges), *Tydeus lambi* (Baker); aus der Gattung *Pronematus*, z. B. *Pronematus ubiquitous* (McGregor);
vii) *Cheyletidae* wie etwa aus der Gattung *Cheyletus*, z. B. *Cheyletus eruditus* (Schrank), *Cheyletus malaccensis* Oudemans;
viii) *Cunaxidae* wie etwa aus der Gattung *Coleoscirus*, z. B. *Coleoscirus simplex* (Ewing), aus der Gattung *Cunaxa*, z. B. *Cunaxa setirostris* (Hermann);
ix) *Erythraeidae* wie etwa aus der Gattung *Balaustium*, z. B. *Balaustium putmani* Smiley, *Balaustium medicagoense* Meyer & Ryke, *Balaustium murorum* (Hermann);
x) *Stigmaeidae* wie etwa aus der Gattung *Agistemus*, z. B. *Agistemus exsertus* Gonzalez; wie etwa aus der Gattung *Zetzellia*, z. B. *Zetzellia mali* (Ewing).

6. Zusammensetzung nach einem der Ansprüche 1 bis 5, wobei es sich bei der Milbenart um eine *Phytoseiid*-Art handelt, vorzugsweise eine *Phytoseiid*-Art, ausgewählt aus *Amblyseius swirskii, Amblyseius aerialis, Amblyseius andersoni, Neoseiulus barkeri, Neoseiulus californicus, Neoseiulus cucumeris, Neoseiulus fallacis, Typhlodromips montdorensis* oder *Amblydromalus limonicus.*

7. Verfahren zur Aufzucht einer Population einer Milbenart, umfassend:
(i) Bereitstellen einer Zusammensetzung nach den Ansprüchen 1 bis 6;
(ii) wobei den Individuen der Milbenpopulation ermöglicht wird, sich von der Nahrungsquelle zu ernähren.

8. Verfahren zur biologischen Schädlingsbekämpfung in einer Kulturpflanze, umfassend das Bereitstellen einer Zusammensetzung nach einem der Ansprüche 1 bis 6 an die Kulturpflanze, wobei die Milbenart als Raubmilbenart ausgewählt wird, wie etwa eine Raubmilbenart ausgewählt aus:
- Mesostigmatiden-Milbenarten, wie etwa ausgewählt aus
i) *Phytoseiidae* wie etwa aus:
- der Unterfamilie der *Amblyseiinae,* wie etwa aus der Gattung *Amblyseius,* z. B. *Amblyseius andersoni, Amblyseius aerialis, Amblyseius swirskii, Amblyseius herbicolus* oder *Amblyseius largoensis,* aus der Gattung *Euseius,* z. B. *Euseius finlandicus, Euseius hibisci, Euseius ovalis, Euseius victoriensis, Euseius stipulatus, Euseius scutalis, Euseius tularensis, Euseius addoensis, Euseius concordis, Euseius ho* oder *Euseius citri,* aus der Gattung *Neoseiulus*, z. B. *Neoseiulus barkeri, Neoseiulus californicus, Neoseiulus cucumeris, Neoseiulus longispinosus, Neoseiulus womersleyi, Neoseiulus idaeus, Neoseiulus anonymus, Neoseiulus paspalivorus, Neoseiulus reductus* oder *Neoseiulus fallacis,* aus der Gattung *Amblydromalus*, z. B. *Amblydromalus limonicus* aus der Gattung *Typhlodromalus*, z. B. *Typhlodromalus aripo, Typhlodromalus laila* oder *Typhlodromalus peregrinus* aus der Gattung *Typhlodromips*, z. B. *Typhlodromips montdorensis,* aus der Gattung *Phytoseiulus*, z. B. *Phytoseiulus persimilis, Phytoseiulus macropilis, Phytoseiulus longipes, Phytoseiulus fragariae;*
- der Unterfamilie der *Typhlodrominae,* wie etwa aus der Gattung *Galendromus,* z. B. *Galendromus occidentalis,* aus der Gattung *Typhlodromus*, z. B. *Typhlodromus pyri, Typhlodromus doreenae* oder *Typhlodromus athiasae;*
*ii) Ascidae* wie etwa aus der Gattung *Proctolaelaps,* wie etwa *Proctolaelaps pygmaeus* (Muller); aus der Gattung *Blattisocius*, z. B. *Blattisocius tarsalis* (Berlese), *Blattisocius keegani* (Fox); aus der Gattung *Lasioseius*, z. B. *Lasioseius fimetorum* Karg, *Lasioseius floridensis* Berlese, *Lasioseius bispinosus* Evans, *Lasioseius dentatus* Fox, *Lasioseius scapulatus* (Kenett), *Lasioseius athiasae* Nawar & Nasr; aus der Gattung *Arctoseius*, z. B. *Arctoseius semiscissus* (Berlese); aus der Gattung *Protogamasellus*, z. B. *Protogamasellus dioscorus* Manson;
*iii) Laelapidae* wie etwa aus der Gattung *Stratiolaelaps*, z. B. *Stratiolaelaps scimitus* (Womersley) (auch in die Gattung *Hypoaspis* eingeordnet); *Geolaelaps,* z. B. *Geolaelaps aculeifer* (Canestrini) (auch in die Gattung *Hypoaspis* eingeordnet); *Androlaelaps*, z. B. *Androlaelaps casalis casalis* (Berlese);
*iv) Macrochelidae* wie etwa aus der Gattung *Macrocheies*, z. B. *Macrocheles robustulus* (Berlese), *Macrocheies muscaedomesticae* (Scopoli), *Macrocheles matrius* (Hull);
*v) Parasitidae* wie etwa aus der Gattung *Pergamasus, z.* B. *Pergamasus quisquiliarum* Canestrini; *Parasitus*, z. B. *Parasitus fimetorum* (Berlese), *Parasitus bituberosus* Karg;
- *Prostigmatiden*-Milbenarten wie etwa aus:
*vi) Tydeidae* wie etwa aus der Gattung *Homeopronematus, z. B. Homeopronematus anconai* (Baker); aus der Gattung *Tydeus,* z. B. *Tydeus lambi* (Baker), *Tydeus caudatus* (Duges), *Tydeus lambi* (Baker); aus der Gattung *Pronematus*, z. B. *Pronematus ubiquitous* (McGregor);
*vii) Cheyletidae* wie etwa aus der Gattung *Cheyletus*, z. B. *Cheyletus eruditus* (Schrank), *Cheyletus malaccensis* Oudemans;
*viii) Cunaxidae* wie etwa aus der Gattung *Coleoscirus*, z. B. *Coleoscirus simplex* (Ewing), aus der Gattung *Cunaxa*, z. B. *Cunaxa setirostris* (Hermann);
*ix) Erythraeidae* wie etwa aus der Gattung *Balaustium*, z. B. *Balaustium putmani* Smiley, *Balaustium medicagoense* Meyer & Ryke , *Balaustium murorum* (Hermann);
*x) Stigmaeidae* wie etwa aus der Gattung *Agistemus*, z. B. *Agistemus exsertus* Gonzalez; wie etwa aus der Gattung *Zetzellia*, z. B. *Zetzellia mali* (Ewing).

9. Aufzuchtvorrichtung zur Aufzucht einer Milbenart, wobei die Vorrichtung einen Behälter umfasst, der die Zusammensetzung nach einem der Ansprüche 1 bis 6 enthält, vorzugsweise einen Behälter, der einen Ausgang für mindestens ein bewegliches Lebensstadium der Milbenart umfasst, weiter bevorzugt einen Ausgang, der geeignet ist, eine anhaltende Freisetzung des mindestens einen beweglichen Lebensstadiums bereitzustellen.

10. Verwendung einer Zusammensetzung nach einem der Ansprüche 1 bis 6 für den Kulturpflanzenschutz, wobei die Milbenart als Raubmilbenart ausgewählt ist, wie etwa eine Raubmilbenart, ausgewählt aus:
- Mesostigmatiden-Milbenarten, wie etwa ausgewählt aus
*i) Phytoseiidae,* wie etwa aus:
- der Unterfamilie der *Amblyseiinae,* wie etwa aus der Gattung *Amblyseius,* z. B. *Amblyseius andersoni, Amblyseius aerialis, Amblyseius swirskii, Amblyseius herbicolus* oder *Amblyseius largoensis,* aus der Gattung *Euseius,* z. B. *Euseius finlandicus, Euseius hibisci, Euseius ovalis, Euseius victoriensis, Euseius stipulatus, Euseius scutalis, Euseius tularensis, Euseius addoensis, Euseius concordis, Euseius ho* oder *Euseius citri,* aus der Gattung *Neoseiulus,* z. B. *Neoseiulus barkeri, Neoseiulus californicus, Neoseiulus cucumeris, Neoseiulus longispinosus, Neoseiulus womersleyi, Neoseiulus idaeus, Neoseiulus anonymus, Neoseiulus paspalivorus, Neoseiulus reductus* oder *Neoseiulus fallacis,* aus der Gattung *Amblydromalus,* z. B. *Amblydromalus limonicus* aus der Gattung *Typhlodromalus,* z. B. *Typhlodromalus aripo, Typhlodromalus laila* oder *Typhlodromalus peregrinus* aus der Gattung *Typhlodromips,* z. B. *Typhlodromips montdorensis,* aus der Gattung *Phytoseiulus,* z. B. *Phytoseiulus persimilis, Phytoseiulus macropilis, Phytoseiulus longipes, Phytoseiulus fragariae*;
- der Unterfamilie der *Typhlodrominae,* wie etwa aus der Gattung *Galendromus,* z. B. *Galendromus occidentalis,* aus der Gattung *Typhlodromus,* z. B. *Typhlodromus pyri, Typhlodromus doreenae* oder *Typhlodromus athiasae;*
*ii) Ascidae* wie etwa aus der Gattung *Proctolaelaps,* wie etwa *Proctolaelaps pygmaeus* (Muller); aus der Gattung *Blattisocius, z.* B. *Blattisocius tarsalis* (Berlese), *Blattisocius keegani* (Fox); aus der Gattung *Lasioseius,* z. B. *Lasioseius fimetorum* Karg, *Lasioseius floridensis* Berlese, *Lasioseius bispinosus* Evans, *Lasioseius dentatus* Fox, *Lasioseius scapulatus* (Kenett), *Lasioseius athiasae* Nawar & Nasr; aus der Gattung *Arctoseius,* z. B. *Arctoseius semiscissus* (Berlese); aus der Gattung *Protogamasellus,* z. B. *Protogamasellus dioscorus* Manson;
iii) *Laelapidae* wie etwa aus der Gattung *Stratiolaelaps,* z. B. *Stratiolaelaps scimitus* (Womersley) (auch in die Gattung *Hypoaspis* eingeordnet); *Geolaelaps,* z. B. *Geolaelaps aculeifer* (Canestrini) (auch in die Gattung *Hypoaspis* eingeordnet); *Androlaelaps,* z. B. *Androlaelaps casalis casalis* (Berlese);
iv) *Macrochelidae* wie etwa aus der Gattung *Macrocheles,* z. B. *Macrocheles robustulus* (Berlese), *Macrocheles muscaedomesticae* (Scopoli), *Macrocheles matrius* (Hull);
v) *Parasitidae* wie etwa aus der Gattung *Pergamasus,* z. B. *Pergamasus quisquiliarum* Canestrini; *Parasitus,* z. B. *Parasitus fimetorum* (Berlese), *Parasitus bituberosus* Karg;
- *Prostigmatiden-Milbenarten* wie etwa aus:
vi) *Tydeidae* wie etwa aus der Gattung *Homeopronematus,* z. *B. Homeopronematus anconai* (Baker); aus der Gattung *Tydeus,* z. B. *Tydeus lambi* (Baker), *Tydeus caudatus* (Duges), *Tydeus lambi* (Baker); aus der Gattung *Pronematus,* z. B. *Pronematus ubiquitous* (McGregor);
vii) *Cheyletidae* wie etwa aus der Gattung *Cheyletus,* z. B. *Cheyletus eruditus* (Schrank), *Cheyletus malaccensis* Oudemans;
viii) *Cunaxidae* wie etwa aus der Gattung *Coleoscirus,* z. B. *Coleoscirus simplex* (Ewing), aus der Gattung *Cunaxa,* z. B. *Cunaxa setirostris* (Hermann);
ix) *Erythraeidae* wie etwa aus der Gattung *Balaustium,* z. B. *Balaustium putmani* Smiley, *Balaustium medicagoense* Meyer & Ryke , *Balaustium murorum* (Hermann);
x) *Stigmaeidae* wie etwa aus der Gattung *Agistemus,* z. B. *Agistemus exsertus* Gonzalez; wie etwa aus der Gattung *Zetzellia,* z. B. *Zetzellia mali* (Ewing).

11. Verwendung eines Trägermaterials, umfassend gestapelte Trägerelemente, ausgewählt aus Hirsespelzen, zur Aufzucht einer Population einer Milbenart, vorzugsweise einer Milbenart ausgewählt aus *Mesostigmatiden-Milbenarten* oder *Prostigmatiden-Milbenarten,* wobei die Trägerelemente Schutzvorrichtungen für Milbenindividuen umfassen.

12. Verwendung nach Anspruch 11, wobei die Schutzvorrichtungen Bereiche umfassen, in denen das Trägermaterial des Trägerelements ein Milbenindividuum, wenn es sich in diesem Bereich befindet, von seiner Umgebung in mindestens 3 Richtungen mit orthogonalen oder umgekehrten Beziehungen, vorzugsweise in mindestens 4 solcher Richtungen, am meisten bevorzugt in mindestens 5 solcher Richtungen, schützt.

13. Verwendung gemäß einem der Ansprüche 11 bis 12, wobei die Schutzvorrichtungen Hohlräume umfassen, wie etwa Hohlräume, die durch Buchten, Vertiefungen, Poren, Kammern, Aussparungen, Nischen, Gruben, Taschen, Rohre und ähnliche Strukturen gebildet werden.

14. Verwendung nach einem der Ansprüche 11 bis 13, wobei die Hirsespelzen von Hirse stammen, die ausgewählt ist aus Perlhirse oder Bajra (*Pennisetum glaucum*), Kolbenhirse (*Setaria italica*), Proso-Hirse, gemeiner Hirse, Rispenhirse, Schweinehirse oder weißer Hirse (*Panicum miliaceum*), Fingerhirse (*Eleusine coracana*), indischer Stallhirse oder Sawahirse (*Echinochloa frumentacea*), Hühnerhirse (*Echinochloa esculenta*), Kodohirse (*Paspalum scrobiculatum*), Zwerghirse (*Panicum sumatrense*), Guineahirse (*Brachiaria deflexa* = *Urochloa deflexa*), Braunhirse (*Urochloa ramosa* = *Brachiaria ramosa* = *Panicum ramosum*), Teff (*Eragrostis tef*), Foniohirse (*Digitaria exilis*), Sorghum (*Sorghum* spp.) oder Hiobstränengras (*Coix lacrima-jobi*).

15. Verwendung nach den Ansprüchen 11 bis 14, wobei es sich bei der Milbenart um eine Raubmilbenart handelt, wie etwa eine Raubmilbenart, ausgewählt aus:
- Mesostigmatiden-Milbenarten, wie etwa ausgewählt aus
*i) Phytoseiidae,* wie etwa aus:
- der Unterfamilie der *Amblyseiinae,* wie etwa aus der Gattung *Amblyseius,* z. B. *Amblyseius andersoni, Amblyseius aerialis, Amblyseius swirskii, Amblyseius herbicolus* oder *Amblyseius largoensis,* aus der Gattung *Euseius,* z. B. *Euseius finlandicus, Euseius hibisci, Euseius ovalis, Euseius victoriensis, Euseius stipulatus, Euseius scutalis, Euseius tularensis, Euseius addoensis, Euseius concordis, Euseius ho* oder *Euseius citri,* aus der Gattung *Neoseiulus,* z. B. *Neoseiulus barkeri, Neoseiulus californicus, Neoseiulus cucumeris, Neoseiulus longispinosus, Neoseiulus womersleyi, Neoseiulus idaeus, Neoseiulus anonymus, Neoseiulus paspalivorus, Neoseiulus reductus* oder *Neoseiulus fallacis,* aus der Gattung *Amblydromalus,* z. B. *Amblydromalus limonicus* aus der Gattung *Typhlodromalus,* z. B. *Typhlodromalus aripo, Typhlodromalus laila* oder *Typhlodromalus peregrinus* aus der Gattung *Typhlodromips,* z. B. *Typhlodromips montdorensis,* aus der Gattung *Phytoseiulus,* z. B. *Phytoseiulus persimilis, Phytoseiulus macropilis, Phytoseiulus longipes, Phytoseiulus fragariae*;
- der Unterfamilie der *Typhlodrominae,* wie etwa aus der Gattung *Galendromus,* z. B. *Galendromus occidentalis,* aus der Gattung *Typhlodromus,* z. B. *Typhlodromus pyri, Typhlodromus doreenae* oder *Typhlodromus athiasae*;
*ii) Ascidae* wie etwa aus der Gattung *Proctolaelaps,* wie etwa *Proctolaelaps pygmaeus* (Muller); aus der Gattung *Blattisocius,* z. B. *Blattisocius tarsalis* (Berlese), *Blattisocius keegani* (Fox); aus der Gattung *Lasioseius,* z. B. *Lasioseius fimetorum* Karg, *Lasioseius floridensis* Berlese, *Lasioseius bispinosus* Evans, *Lasioseius dentatus* Fox, *Lasioseius scapulatus* (Kenett), *Lasioseius athiasae* Nawar & Nasr; aus der Gattung *Arctoseius,* z. B. *Arctoseius semiscissus* (Berlese); aus der Gattung *Protogamasellus,* z. B. *Protogamasellus dioscorus* Manson;
iii) *Laelapidae* wie etwa aus der Gattung *Stratiolaelaps,* z. B. *Stratiolaelaps scimitus* (Womersley) (auch in die Gattung *Hypoaspis* eingeordnet); *Geolaelaps,* z. B. *Geolaelaps aculeifer* (Canestrini) (auch in die Gattung *Hypoaspis* eingeordnet); *Androlaelaps,* z. B. *Androlaelaps casalis casalis* (Berlese);
iv) *Macrochelidae* wie etwa aus der Gattung *Macrocheles,* z. B. *Macrocheles robustulus* (Berlese), *Macrocheles muscaedomesticae* (Scopoli), *Macrocheles matrius* (Hull);
v) *Parasitidae* wie etwa aus der Gattung *Pergamasus,* z. B. *Pergamasus quisquiliarum* Canestrini; *Parasitus,* z. B. *Parasitus fimetorum* (Berlese), *Parasitus bituberosus* Karg;
- *Prostigmatiden-Milbenarten* wie etwa aus:
vi) *Tydeidae* wie etwa aus der Gattung *Homeopronematus, z. B. Homeopronematus anconai* (Baker); aus der Gattung *Tydeus,* z. B. *Tydeus lambi* (Baker), *Tydeus caudatus* (Duges), *Tydeus lambi* (Baker); aus der Gattung *Pronematus,* z. B. *Pronematus ubiquitous* (McGregor);
vii) *Cheyletidae* wie etwa aus der Gattung *Cheyletus,* z. B. *Cheyletus eruditus* (Schrank), *Cheyletus malaccensis* Oudemans;
viii) *Cunaxidae* wie etwa aus der Gattung *Coleoscirus,* z. B. *Coleoscirus simplex* (Ewing), aus der Gattung *Cunaxa,* z. B. *Cunaxa setirostris* (Hermann);
ix) *Erythraeidae* wie etwa aus der Gattung *Balaustium,* z. B. *Balaustium putmani* Smiley, *Balaustium medicagoense* Meyer & Ryke, *Balaustium murorum* (Hermann);
x) *Stigmaeidae* wie etwa aus der Gattung *Agistemus,* z. B. *Agistemus exsertus* Gonzalez; wie etwa aus der Gattung *Zetzellia,* z. B. *Zetzellia mali* (Ewing).

## Revendications

1. Composition d'acariens, comprenant :
- une population d'individus (1) issus d'une espèce d'acarien, de préférence une espèce d'acarien choisie parmi une espèce d'acarien de type *Mesostigmata* ou une espèce d'acarien de type *Prostigmata ;*
- une source d'alimentation pour les individus acariens ; et
- un support pour les individus de l'espèce d'acarien comprenant des éléments de support empilés choisis parmi des cosses de millet ;
où les éléments de support comprennent des abris pour les individus acariens.

2. Composition selon la revendication 1, dans laquelle les abris comprennent des zones dans lesquelles le matériau de l'élément de support abrite un individu acarien, lorsqu'il est situé dans cette zone, de son environnement selon au moins 3 directions ayant des relations orthogonales ou inversées, préférablement selon au moins 4 parmi de telles directions, tout préférablement selon au moins 5 parmi de telles directions.

3. Composition selon l'une quelconque des revendications 1-2, dans laquelle les abris comprennent des vides, tels que les vides formés par des criques, des renfoncements, des pores, des chambres, des cavités, des niches, des puits, des poches, des tubes et des structures similaires.

4. Composition selon l'une quelconque des revendications 1-3, dans laquelle les cosses de millet sont issues de millet choisi parmi le mil à chandelle ou mil (*Pennisetum glaucum*) ; la sétaire d'Italie (*Setaria italica*) ; le panic millet, le millet commun, le millet des oiseaux, le millet cultivé ou le millet blanc (*Panicum miliaceum*) ; l'éleusine (*Eleusine coracana*), le pied-de-coq cultivé ou le millet japonais (*Echinochloa frumentacea*) ; le millet du Japon (*Echinochloa esculenta*) ; le millet Kodo (*Paspalum scrobiculatum*) ; le petit millet (*Panicum sumatrense*) ; le fonio à grosses graines (*Brachiaria deflexa* = *Urochloa deflexa*) ; le millet rampant (*Urochloa ramosa* = *Brachiaria ramosa* = *Panicum ramosum*), le tef (*Eragrostis tef*), le fonio blanc (*Digitaria exilis*), le sorgho (*Sorghum* spp.) ou la Larme de Jacob (*Coix lacryma-jobi*).

5. Composition selon l'une quelconque des revendications précédentes, dans laquelle l'espèce d'acarien est choisie parmi :
- une espèce d'acarien de type *Mesostigmata* telle que choisie parmi :
i) *Phytoseiidae* tel que parmi :
- la sous-famille des *Amblyseiinae,* tel que du genre *Amblyseius,* par exemple *Amblyseius andersoni, Amblyseius aerialis, Amblyseius swirskii, Amblyseius herbicolus* ou *Amblyseius largoensis,* du genre *Euseius* par exemple *Euseius finlandicus, Euseius hibisci, Euseius ovalis, Euseius victoriensis, Euseius stipulatus, Euseius scutalis, Euseius tularensis, Euseius addoensis, Euseius concordis, Euseius ho* ou *Euseius citri,* du genre *Neoseiulus* par exemple *Neoseiulus barkeri, Neoseiulus californicus, Neoseiulus cucumeris, Neoseiulus longispinosus, Neoseiulus womersleyi, Neoseiulus idaeus, Neoseiulus anonymus, Neoseiulus paspalivorus, Neoseiulus reductus* ou *Neoseiulus fallacis,* du genre *Amblydromalus* par exemple *Amblydromalus limonicus,* du genre *Typhlodromalus* par exemple *Typhlodromalus aripo, Typhlodromalus laila* ou *Typhlodromalus peregrinus,* du genre *Typhlodromips* par exemple *Typhlodromips montdorensis,* du genre *Phytoseiulus,* par exemple *Phytoseiulus persimilis, Phytoseiulus macropilis, Phytoseiulus longipes, Phytoseiulus fragariae ;*
- la sous-famille des *Typhlodrominae,* tel que du genre *Galendromus* par exemple *Galendromus occidentalis,* du genre *Typhlodromus* par exemple *Typhlodromus pyri, Typhlodromus doreenae ou Typhlodromus athiasae ;*
ii) *Ascidae* tel que du genre *Proctolaelaps,* tel que *Proctolaelaps pygmaeus* (Muller) ; du genre *Blattisocius* par exemple *Blattisocius tarsalis* (Berlese), *Blattisocius keegani* (Fox) ; du genre *Lasioseius* par exemple *Lasioseius fimetorum* Karg, *Lasioseius floridensis* Berlese, *Lasioseius bispinosus* Evans, *Lasioseius dentatus* Fox, *Lasioseius scapulatus* (Kenett), *Lasioseius athiasae* Nawar & Nasr ; du genre *Arctoseius* par exemple *Arctoseius semiscissus* (Berlese) ; du genre *Protogamasellus* par exemple *Protogamasellus dioscorus* Manson ;
iii) *Laelapidae* tel que du genre *Stratiolaelaps* par exemple *Stratiolaelaps scimitus* (Womersley) (également placé dans le genre *Hypoaspis*) ; *Geolaelaps* par exemple *Geolaelaps aculeifer* (Canestrini) (également placé dans le genre *Hypoaspis*) ; *Androlaelaps* par exemple *Androlaelaps casalis casalis* (Berlese) ;
iv) *Macrochelidae* tel que du genre *Macrocheles* par exemple *Macrocheles robustulus* (Berlese), *Macrocheles muscaedomesticae* (Scopoli), *Macrocheles matrius* (Hull) ;
v) *Parasitidae* tel que du genre *Pergamasus* par exemple *Pergamasus quisquiliarum* Canestrini ; *Parasitus* par exemple *Parasitus fimetorum* (Berlese), *Parasitus bituberosus* Karg ;
- une espèce d'acarien de type *Prostigmata,* tel que parmi
vi) *Tydeidae* tel que du genre *Homeopronematus* par exemple *Homeopronematus anconai* (Baker) ; du genre *Tydeus* par exemple *Tydeus lambi* (Baker), *Tydeus caudatus* (Dugés), *Tydeus lambi* (Baker) ; du genre *Pronematus* par exemple *Pronematus ubiquitous* (McGregor) ;
vii) *Cheyletidae* tel que du genre *Cheyletus* par exemple *Cheyletus eruditus* (Schrank), *Cheyletus malaccensis* Oudemans ;
viii) *Cunaxidae* tel que du genre *Coleoscirus* par exemple *Coleoscirus simplex* (Ewing), du genre *Cunaxa* par exemple *Cunaxa setirostris* (Hermann) ;
ix) *Erythraeidae* tel que du genre *Balaustium* par exemple *Balaustium putmani* Smiley, *Balaustium medicagoense* Meyer & Ryke, *Balaustium murorum* (Hermann) ;
x) *Stigmaeidae* tel que du genre *Agistemus* par exemple *Agistemus exsertus* Gonzalez ; tel que du genre *Zetzellia* par exemple *Zetzelliamali* (Ewing).

6. Composition selon l'une quelconque des revendications 1-5, dans laquelle l'espèce d'acarien est une espèce de *Phytoseiidae,* préférablement une espèce de *Phytoseiidae* choisie parmi *Amblyseius swirskii, Amblyseius aerialis, Amblyseius andersoni, Neoseiulus barkeri, Neoseiulus californicus, Neoseiulus cucumeris, Neoseiulus fallacis, Typhlodromips montdorensis* ou *Amblydromalus limonicus.*

7. Méthode d'élevage d'une population d'une espèce d'acarien, comprenant les étapes consistant à :
(i) mettre à disposition une composition selon les revendications 1-6 ;
(ii) permettre aux individus de la population d'acariens de se nourrir sur la source alimentaire.

8. Méthode de contrôle biologique de nuisibles dans une culture, comprenant la mise à disposition pour ladite culture d'une composition selon l'une quelconque des revendications 1-6, dans laquelle l'espèce d'acarien est choisie parmi une espèce d'acarien prédateur, telle qu'une espèce d'acarien prédateur choisie parmi
- une espèce d'acarien de type *Mesostigmata* telle que choisie parmi :
i) *Phytoseiidae* tel que parmi :
- la sous-famille des *Amblyseiinae,* tel que du genre *Amblyseius,* par exemple *Amblyseius andersoni, Amblyseius aerialis, Amblyseius swirskii, Amblyseius herbicolus* ou *Amblyseius largoensis,* du genre *Euseius* par exemple *Euseius finlandicus, Euseius hibisci, Euseius ovalis, Euseius victoriensis, Euseius stipulatus, Euseius scutalis, Euseius tularensis, Euseius addoensis, Euseius concordis, Euseius ho* ou *Euseius citri,* du genre *Neoseiulus* par exemple *Neoseiulus barkeri, Neoseiulus californicus, Neoseiulus cucumeris, Neoseiulus longispinosus, Neoseiulus womersleyi, Neoseiulus idaeus, Neoseiulus anonymus, Neoseiulus paspalivorus, Neoseiulus reductus* ou *Neoseiulus fallacis,* du genre *Amblydromalus* par exemple *Amblydromalus limonicus,* du genre *Typhlodromalus* par exemple *Typhlodromalus aripo, Typhlodromalus laila* ou *Typhlodromalus peregrinus,* du genre *Typhlodromips* par exemple *Typhlodromips montdorensis,* du genre *Phytoseiulus,* par exemple *Phytoseiulus persimilis, Phytoseiulus macropilis, Phytoseiulus longipes, Phytoseiulus fragariae ;*
- la sous-famille des *Typhlodrominae,* tel que du genre *Galendromus* par exemple *Galendromus occidentalis,* du genre *Typhlodromus* par exemple *Typhlodromus pyri, Typhlodromus doreenae ou Typhlodromus athiasae ;*
ii) *Ascidae* tel que du genre *Proctolaelaps,* tel que *Proctolaelaps pygmaeus* (Muller) ; du genre *Blattisocius* par exemple *Blattisocius tarsalis* (Berlese), *Blattisocius keegani* (Fox) ; du genre *Lasioseius* par exemple *Lasioseius fimetorum* Karg, *Lasioseius floridensis* Berlese, *Lasioseius bispinosus* Evans, *Lasioseius dentatus* Fox, *Lasioseius scapulatus* (Kenett), *Lasioseius athiasae* Nawar & Nasr ; du genre *Arctoseius* par exemple *Arctoseius semiscissus* (Berlese) ; du genre *Protogamasellus* par exemple *Protogamasellus dioscorus* Manson ;
iii) *Laelapidae* tel que du genre *Stratiolaelaps* par exemple *Stratiolaelaps scimitus* (Womersley) (également placé dans le genre *Hypoaspis) ; Geolaelaps* par exemple *Geolaelaps aculeifer* (Canestrini) (également placé dans le genre *Hypoaspis*) *; Androlaelaps* par exemple *Androlaelaps casalis casalis* (Berlese) ;
iv) *Macrochelidae* tel que du genre *Macrocheles* par exemple *Macrocheles robustulus* (Berlese), *Macrocheles muscaedomesticae* (Scopoli), *Macrocheles matrius* (Hull) ;
v) *Parasitidae* tel que du genre *Pergamasus* par exemple *Pergamasus quisquiliarum* Canestrini ; *Parasitus* par exemple *Parasitus fimetorum* (Berlese), *Parasitus bituberosus* Karg ;
- une espèce d'acarien de type *Prostigmata,* tel que parmi
vi) *Tydeidae* tel que du genre *Homeopronematus* par exemple *Homeopronematus anconai* (Baker) ; du genre *Tydeus* par exemple *Tydeus lambi* (Baker), *Tydeus caudatus* (Dugés), *Tydeus lambi* (Baker) ; du genre *Pronematus* par exemple *Pronematus ubiquitous* (McGregor) ;
vii) *Cheyletidae* tel que du genre *Cheyletus* par exemple *Cheyletus eruditus* (Schrank), *Cheyletus malaccensis* Oudemans ;
viii) *Cunaxidae* tel que du genre *Coleoscirus* par exemple *Coleoscirus simplex* (Ewing), du genre *Cunaxa* par exemple *Cunaxa setirostris* (Hermann) ;
ix) *Erythraeidae* tel que du genre *Balaustium* par exemple *Balaustium putmani* Smiley, *Balaustium medicagoense* Meyer & Ryke, *Balaustium murorum* (Hermann) ;
x) *Stigmaeidae* tel que du genre *Agistemus* par exemple *Agistemus exsertus* Gonzalez ; tel que du genre *Zetzellia* par exemple *Zetzelliamali* (Ewing).

9. Dispositif d'élevage destiné à l'élevage d'une espèce d'acarien, ledit dispositif comprenant un conteneur accueillant la composition selon l'une quelconque des revendications 1-6, préférablement un conteneur comprenant une sortie pour au moins un stade de vie mobile de l'espèce d'acarien, plus préférablement une sortie convenable pour la fourniture d'une libération prolongée dudit au moins un stade de vie mobile.

10. Utilisation, pour la protection de cultures, d'une composition selon l'une quelconque des revendications 1-6, dans laquelle l'espèce d'acarien est choisie parmi une espèce d'acarien prédateur, telle qu'une espèce d'acarien prédateur choisie parmi
- une espèce d'acarien de type *Mesostigmata* telle que choisie parmi :
i) *Phytoseiidae* tel que parmi :
- la sous-famille des *Amblyseiinae,* tel que du genre *Amblyseius,* par exemple *Amblyseius andersoni, Amblyseius aerialis, Amblyseius swirskii, Amblyseius herbicolus* ou *Amblyseius largoensis,* du genre *Euseius* par exemple *Euseius finlandicus, Euseius hibisci, Euseius ovalis, Euseius victoriensis, Euseius stipulatus, Euseius scutalis, Euseius tularensis, Euseius addoensis, Euseius concordis, Euseius ho* ou *Euseius citri,* du genre *Neoseiulus* par exemple *Neoseiulus barkeri, Neoseiulus californicus, Neoseiulus cucumeris, Neoseiulus longispinosus, Neoseiulus womersleyi, Neoseiulus idaeus, Neoseiulus anonymus, Neoseiulus paspalivorus, Neoseiulus reductus* ou *Neoseiulus fallacis,* du genre *Amblydromalus* par exemple *Amblydromalus limonicus,* du genre *Typhlodromalus* par exemple *Typhlodromalus aripo, Typhlodromalus laila* ou *Typhlodromalus peregrinus,* du genre *Typhlodromips* par exemple *Typhlodromips montdorensis,* du genre *Phytoseiulus,* par exemple *Phytoseiulus persimilis, Phytoseiulus macropilis, Phytoseiulus longipes, Phytoseiulus fragariae ;*
- la sous-famille des *Typhlodrominae,* tel que du genre *Galendromus* par exemple *Galendromus occidentalis,* du genre *Typhlodromus* par exemple *Typhlodromus pyri, Typhlodromus doreenae ou Typhlodromus athiasae ;*
ii) *Ascidae* tel que du genre *Proctolaelaps,* tel que *Proctolaelaps pygmaeus* (Muller) ; du genre *Blattisocius* par exemple *Blattisocius tarsalis* (Berlese), *Blattisocius keegani* (Fox) ; du genre *Lasioseius* par exemple *Lasioseius fimetorum* Karg, *Lasioseius floridensis* Berlese, *Lasioseius bispinosus* Evans, *Lasioseius dentatus* Fox, *Lasioseius scapulatus* (Kenett), *Lasioseius athiasae* Nawar & Nasr ; du genre *Arctoseius* par exemple *Arctoseius semiscissus* (Berlese) ; du genre *Protogamasellus* par exemple *Protogamasellus dioscorus* Manson ;
iii) *Laelapidae* tel que du genre *Stratiolaelaps* par exemple *Stratiolaelaps scimitus* (Womersley) (également placé dans le genre *Hypoaspis) ; Geolaelaps* par exemple *Geolaelaps aculeifer* (Canestrini) (également placé dans le genre *Hypoaspis*) ; *Androlaelaps* par exemple *Androlaelaps casalis casalis* (Berlese) ;
iv) *Macrochelidae* tel que du genre *Macrocheles* par exemple *Macrocheles robustulus* (Berlese), *Macrocheles muscaedomesticae* (Scopoli), *Macrocheles matrius* (Hull) ;
v) *Parasitidae* tel que du genre *Pergamasus* par exemple *Pergamasus quisquiliarum* Canestrini ; *Parasitus* par exemple *Parasitus fimetorum* (Berlese), *Parasitus bituberosus* Karg ;
- une espèce d'acarien de type *Prostigmata,* tel que parmi
vi) *Tydeidae* tel que du genre *Homeopronematus* par exemple *Homeopronematus anconai* (Baker) ; du genre *Tydeus* par exemple *Tydeus lambi* (Baker), *Tydeus caudatus* (Dugés), *Tydeus lambi* (Baker) ; du genre *Pronematus* par exemple *Pronematus ubiquitous* (McGregor) ;
vii) *Cheyletidae* tel que du genre *Cheyletus* par exemple *Cheyletus eruditus* (Schrank), *Cheyletus malaccensis* Oudemans ;
viii) *Cunaxidae* tel que du genre *Coleoscirus* par exemple *Coleoscirus simplex* (Ewing), du genre *Cunaxa* par exemple *Cunaxa setirostris* (Hermann) ;
ix) *Erythraeidae* tel que du genre *Balaustium* par exemple *Balaustium putmani* Smiley, *Balaustium medicagoense* Meyer & Ryke, *Balaustium murorum* (Hermann) ;
x) *Stigmaeidae* tel que du genre *Agistemus* par exemple *Agistemus exsertus* Gonzalez ; tel que du genre *Zetzellia* par exemple *Zetzelliamali* (Ewing).

11. Utilisation d'un matériau de support comprenant des éléments de support empilés choisis parmi des cosses de millet, pour l'élevage d'une population d'une espèce d'acarien, préférablement d'une espèce d'acarien choisie parmi une espèce d'acarien de type *Mesostigmata* ou une espèce d'acarien de type *Prostigmata,* dans laquelle les éléments de support comprennent des abris pour les individus acariens.

12. Utilisation selon la revendication 11, dans laquelle les abris comprennent des zones dans lesquelles le matériau de support de l'élément de support abrite un individu acarien, lorsqu'il est situé dans cette zone, de son environnement selon au moins 3 directions ayant des relations orthogonales ou inversées, préférablement selon au moins 4 parmi de telles directions, tout préférablement selon au moins 5 parmi de telles directions.

13. Utilisation selon l'une quelconque des revendications 11-12, dans laquelle les abris comprennent des vides, tels que les vides formés par des criques, des renfoncements, des pores, des chambres, des cavités, des niches, des puits, des poches, des tubes et des structures similaires.

14. Utilisation selon l'une quelconque des revendications 11-13, dans laquelle les cosses de millet sont issues de millet choisi parmi le mil à chandelle ou mil (*Pennisetum glaucum*) ; la sétaire d'Italie (*Setaria italica*) ; le panic millet, le millet commun, le millet des oiseaux, le millet cultivé ou le millet blanc (*Panicum miliaceum*) ; l'éleusine (*Eleusine coracana*), le pied-de-coq cultivé ou le millet japonais (*Echinochloa frumentacea*) ; le millet du Japon (*Echinochloa esculenta*) ; le millet Kodo (*Paspalum scrobiculatum*) ; le petit millet (*Panicum sumatrensei*) ; le fonio à grosses graines (*Brachiaria deflexa* = *Urochloa deflexa*) ; le millet rampant (*Urochloa ramosa* = *Brachiaria ramosa* = *Panicum ramosum*), le tef (*Eragrostis tef*), le fonio blanc (*Digitaria exilis*), le sorgho (*Sorghum* spp.) ou la Larme de Jacob (*Coix lacryma-jobi*).

15. Utilisation selon les revendications 11-14, dans laquelle l'espèce d'acarien est une espèce d'acarien prédateur, telle qu'une espèce d'acarien prédateur choisie parmi :
- une espèce d'acarien de type *Mesostigmata* telle que choisie parmi :
i) *Phytoseiidae* tel que parmi :
- la sous-famille des *Amblyseiinae,* tel que du genre *Amblyseius,* par exemple *Amblyseius andersoni, Amblyseius aerialis, Amblyseius swirskii, Amblyseius herbicolus* ou *Amblyseius largoensis,* du genre *Euseius* par exemple *Euseius finlandicus, Euseius hibisci, Euseius ovalis, Euseius victoriensis, Euseius stipulatus, Euseius scutalis, Euseius tularensis, Euseius addoensis, Euseius concordis, Euseius ho* ou *Euseius citri,* du genre *Neoseiulus* par exemple *Neoseiulus barkeri, Neoseiulus californicus, Neoseiulus cucumeris, Neoseiulus longispinosus, Neoseiulus womersleyi, Neoseiulus idaeus, Neoseiulus anonymus, Neoseiulus paspalivorus, Neoseiulus reductus* ou *Neoseiulus fallacis,* du genre *Amblydromalus* par exemple *Amblydromalus limonicus,* du genre *Typhlodromalus* par exemple *Typhlodromalus aripo, Typhlodromalus laila* ou *Typhlodromalus peregrinus,* du genre *Typhlodromips* par exemple *Typhlodromips montdorensis,* du genre *Phytoseiulus,* par exemple *Phytoseiulus persimilis, Phytoseiulus macropilis, Phytoseiulus longipes, Phytoseiulus fragariae ;*
- la sous-famille des *Typhlodrominae,* tel que du genre *Galendromus* par exemple *Galendromus occidentalis,* du genre *Typhlodromus* par exemple *Typhlodromus pyri, Typhlodromus doreenae ou Typhlodromus athiasae ;*
ii) *Ascidae* tel que du genre *Proctolaelaps,* tel que *Proctolaelaps pygmaeus* (Muller) ; du genre *Blattisocius* par exemple *Blattisocius tarsalis* (Berlese), *Blattisocius keegani* (Fox) ; du genre *Lasioseius* par exemple *Lasioseius fimetorum* Karg, *Lasioseius floridensis* Berlese, *Lasioseius bispinosus* Evans, *Lasioseius dentatus* Fox, *Lasioseius scapulatus* (Kenett), *Lasioseius athiasae* Nawar & Nasr ; du genre *Arctoseius* par exemple *Arctoseius semiscissus* (Berlese) ; du genre *Protogamasellus* par exemple *Protogamasellus dioscorus* Manson ;
iii) *Laelapidae* tel que du genre *Stratiolaelaps* par exemple *Stratiolaelaps scimitus* (Womersley) (également placé dans le genre *Hypoaspis) ; Geolaelaps* par exemple *Geolaelaps aculeifer* (Canestrini) (également placé dans le genre *Hypoaspis*) ; *Androlaelaps* par exemple *Androlaelaps casalis casalis* (Berlese) ;
iv) *Macrochelidae* tel que du genre *Macrocheles* par exemple *Macrocheles robustulus* (Berlese), *Macrocheles muscaedomesticae* (Scopoli), *Macrocheles matrius* (Hull) ;
v) *Parasitidae* tel que du genre *Pergamasus* par exemple *Pergamasus quisquiliarum* Canestrini ; *Parasitus* par exemple *Parasitus fimetorum* (Berlese), *Parasitus bituberosus* Karg ;
- une espèce d'acarien de type *Prostigmata,* tel que parmi
vi) *Tydeidae* tel que du genre *Homeopronematus* par exemple *Homeopronematus anconai* (Baker) ; du genre *Tydeus* par exemple *Tydeus lambi* (Baker), *Tydeus caudatus* (Dugés), *Tydeus lambi* (Baker) ; du genre *Pronematus* par exemple *Pronematus ubiquitous* (McGregor) ;
vii) *Cheyletidae* tel que du genre *Cheyletus* par exemple *Cheyletus eruditus* (Schrank), *Cheyletus malaccensis* Oudemans ;
viii) *Cunaxidae* tel que du genre *Coleoscirus* par exemple *Coleoscirus simplex* (Ewing), du genre *Cunaxa* par exemple *Cunaxa setirostris* (Hermann) ;
ix) *Erythraeidae* tel que du genre *Balaustium* par exemple *Balaustium putmani* Smiley, *Balaustium medicagoense* Meyer & Ryke, *Balaustium murorum* (Hermann) ;
x) *Stigmaeidae* tel que du genre *Agistemus* par exemple *Agistemus exsertus* Gonzalez ; tel que du genre *Zetzellia* par exemple *Zetzelliamali* (Ewing).
